# EUROPÄISCHE PATENTSCHRIFT

(11) **EP 2 925 284 B1**
(45) Veröffentlichungstag und Bekanntmachung des Hinweises auf die Patenterteilung: **28.06.2017**
(21) Anmeldenummer: 13795485.5
(22) Anmeldetag: 25.11.2013
(51) Int. Cl.: A61K 8/35, A61K 8/41, A61K 8/44, A61K 8/81, A61K 8/06

(54) **FARBGLANZMITTEL MIT SPEZIELLEN KATIONISCHEN FARBSTOFFEN, TENSIDEN UND POLYMEREN**
DYE GLOSS AGENT WITH SPECIAL CATIONIC PIGMENTS, TENSIDES AND POLYMERS
BRILLANTINE DOTÉE DE COLORANTS CATIONIQUES, DE TENSIO-ACTIFS ET DE POLYMÈRES SPÉCIAUX

(30) Priorität: 30.11.2012 DE 102012221987
(43) Veröffentlichungstag der Anmeldung: 07.10.2015
(73) Patentinhaber: Henkel AG & Co. KGaA, 40589 Düsseldorf (DE)
(72) Erfinder: GOUTSIS, Konstantin, 41363 Jüchen (DE); WESER, Gabriele, 41472 Neuss (DE)
(86) Internationale Anmeldenummer: PCT/EP2013/074564
(87) Internationale Veröffentlichungsnummer: WO 2014/082948

(56) Entgegenhaltungen:
- EP-A1- 0 852 136
- DE-A1- 10 144 881
- US-A- 5 891 200
- US-A1- 2006 100 114

## Beschreibung

Die vorliegende Erfindung liegt im Gebiet der Kosmetik und betrifft Mittel zum Färben von keratinischen Fasern, insbesondere menschlichen Haaren, welche spezielle Kombinationen aus kationischen Anthrachinonfarbstoffen mit weiteren kationischen Farbstoffen, mit amphoteren bzw. zwitterionischen Tensiden und mit kationischen Polymeren enthalten.

Für das Färben von keratinischen Fasern kommen im allgemeinen entweder direktziehende Farbstoffe oder Oxidationsfarbstoffe zur Anwendung. Mit Oxidationsfarbstoffen lassen sich zwar intensive Färbungen mit guten Echtheitseigenschaften erzielen, die Entwicklung der Farbe geschieht jedoch im allgemeinen unter dem Einfluss von Oxidationsmitteln wie z. B. H₂O₂, was in einigen Fällen Schädigungen der Faser zur Folge haben kann. Des Weiteren können einige Oxidationsfarbstoffvorprodukte bzw. bestimmte Mischungen von Oxidationsfarbstoffvorprodukten bei Personen mit empfindlicher Haut sensibilisierend wirken. Direktziehende Farbstoffe werden unter schonenderen Bedingungen appliziert. Ihr Nachteil liegt jedoch darin, dass die Färbungen häufig nur über unzureichende Echtheitseigenschaften verfügen, insbesondere bei der Haarwäsche, aber auch gegenüber äußeren Einflüssen, wie Sonnenlicht oder reaktiven Umweltchemikalien, wie beispielsweise Schwimmbadwasser. Solche Färbungen sind auch gegen Shampoonieren in der Regel deutlich empfindlicher als die oxidativen Färbungen, so dass dann sehr viel schneller eine vielfach unerwünschte Nuancenverschiebung oder gar eine sichtbare "Entfärbung" eintritt.

Neben einer schönen Haarfarbe wünscht sich der Verbraucher auch glänzendes Haar. Glänzendes Haar wirkt attraktiv und gesund, die Frisur wird als gepflegt und vital wahrgenommen. In vielen Fällen hat der Verbraucher mehrere Wünsche, die er zeitgleich erfüllen möchte, d.h. er wünscht sich eine attraktive Haarfärbung, und gleichzeitig wünscht er sich glänzendes Haar. Aus praktischen Gründen möchte er sein Haar hierfür nur einem Behandlungsverfahren unterziehen.

Aus dem Stand der Technik sind bereits diverse Methoden bekannt, das Haar in den verschiedensten Nuancen zu färben. Eine große Anzahl diverser Rezepturen stellt beispielsweise CA 2613049 A1 zur Verfügung. US-A-2006/0100114 thematisiert farbverstärkende Shampoos, die hinsichtlich ihres Glanzes sowie ihrer Weichheit, Frisierbarkeit und Farbverstärkung optimiert sein sollen. Beispiele 1-3 offenbaren Haarfärbemittel enthaltend kationische, direktziehende Farbstoffe (z.B. Basic red 76 und Basic red 51), amphotere Tenside (Cocoamidopropylbetaine) und kationische Polymere (z.B. Polyquaternium-7 oder Polyquaternium-11). Nach wie vor besteht jedoch Bedarf an neuen Färbemitteln, die gleichzeitig eine intensive Färbung und einen außergewöhnlich hohen Glanz erzeugen.

Insbesondere besteht hierbei Bedarf an neuen Färbmitteln, die einen lang anhaltenden Glanz zu erzeugen vermögen. Die mit diesen Mitteln gefärbten Haare sollen ihren Glanz nicht nach einer, bzw. spätestens nach einigen wenigen Wäschen verlieren, sondern ihr Glanz soll auch noch mehreren Haarwäschen noch wahrnehmbar sein.

Aufgabe der vorliegenden Erfindung war daher die Bereitstellung von neuen direktziehenden Färbemitteln, die das Haar intensiv färben und ihm gleichzeitig einen sehr starken Glanz verleihen. Der Glanz der mit diesen Mitteln erzeugten Färbungen soll hierbei lang anhaltend sein und mehrere Haarwäschen überdauern.

Überraschenderweise konnte nun gefunden werden, dass Mittel, die eine Kombination aus einem speziellen kationischen Anthrachinonderivat mit einem weiteren kationischen Farbstoff, mit einem amphoteren bzw. zwitterionischen Tensid und mit einem kationischen Polymer enthalten, das Haar intensiv färben und ihm gleichzeitig einen starken Glanz verleihen. Darüber hinaus ist der mit diesen Mitteln erzeugte Glanz lang anhaltend und auch nach mehreren Haarwäschen bzw. Shampoo-Behandlungen noch sichtbar.

Gleichzeitig erfüllen diese Mittel auch alle anderen an die Färbemittel für keratinische Fasern gestellten Ansprüche im Hinblick auf ihre anwendungstechnischen Eigenschaften, ihre Echtheitseigenschaften, ihr toxikologisches Profil, ihre industrielle Herstellbarkeit und ihre Lagerstabilität.

Ein erster Gegenstand der vorliegenden Erfindung ist ein Mittel zum Färben von keratinischen Fasern, insbesondere menschlichen Haaren, enthaltend in einem kosmetischen Träger
(a) mindestens ein kationisches Anthrachinonderivat der Formel (I) 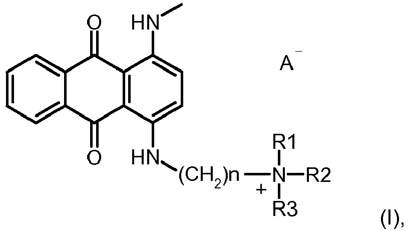 worin
   - R1, R2, R3: unabhängig voneinander für eine C₁-C₆-Alkylgruppe stehen,
   - n: für eine ganze Zahl von 2 bis 8 steht und
   - A⁻: für ein physiologisch verträgliches Anion steht,
(b) mindestens einen weiteren kationischen direktziehenden Farbstoff, der von Formel (I) verschieden ist,
(c) mindestens ein amphoteres und/oder zwitterionisches Tensid und
(d) mindestens ein kationisches Polymer.

Unter keratinischen Fasern oder auch Keratinfasern sind dabei Pelze, Wolle, Federn und insbesondere menschliche Haare zu verstehen. Obwohl die erfindungsgemäßen Mittel in erster Linie zum Färben von Keratinfasern geeignet sind, steht prinzipiell einer Verwendung auch auf anderen Gebieten nichts entgegen.

Unter dem erfindungsgemäß verwendeten Begriff "Mittel zum Färben" von Keratinfasern werden Färbemittel verstanden, die die Keratinfasern auf Basis von direktziehenden Farbstoffen färben. Zusätzlich können diese Mittel auch Oxidationsfarbstoffvorprodukte, sogenannte Entwickler und Kupplerkomponenten enthalten. Entwickler und Kuppler diffundieren getrennt in die Keratinfaser hinein und bilden unter dem Einfluss von einem Alkalisierungsmittel (z.B. Ammoniak) und einem Oxidationsmittel (meist Wasserstoffperoxid) in chemischer Reaktion miteinander die eigentlichen Farbstoffe aus. Enthalten die Mittel zum Färben von Haaren ausschließlich direktziehende Farbstoffe, so werden sie gefärbt, ohne hierbei gleichzeitig aufgehellt zu werden. Enthalten die Mittel jedoch zusätzlich Entwickler, Kuppler und/oder Oxidationsmittel, so werden die Keratinfasern - bedingt durch das zusätzlich enthaltene Oxidationsmittel - zusätzlich aufgehellt. Eine aufhellende Färbung ist im Sinne der vorliegenden Erfindung von der Definition der Färbung mit umfasst.

Die erfindungsgemäßen Mittel enthalten die Wirkstoffe in einem kosmetischen Träger, bevorzugt in einem geeigneten wässrigen, alkoholischen oder wässrig-alkoholischen Träger. Zum Zwecke der Haarfärbung sind solche Träger beispielsweise Cremes, Emulsionen, Gele oder auch tensidhaltige schäumende Lösungen, wie beispielsweise Shampoos, Schaumaerosole, Schaumformulierungen oder andere Zubereitungen, die für die Anwendung auf dem Haar geeignet sind. Es ist aber auch denkbar, die erfindungsgemäßen Mittel in eine pulverförmige oder auch tablettenförmige Formulierung zu integrieren.

Unter wässrig-alkoholischen Lösungen sind im Sinne der vorliegenden Erfindung wässrige Lösungen enthaltend 3 bis 70 Gew.-% eines C₁-C₄-Alkohols, insbesondere Ethanol bzw. Isopropanol, zu verstehen. Die erfindungsgemäßen Mittel können zusätzlich weitere organische Lösemittel, wie beispielsweise Methoxybutanol, Benzylalkohol, Ethyldiglykol oder 1,2-Propylenglykol, enthalten. Bevorzugt sind dabei alle wasserlöslichen organischen Lösemittel. Der wässriger Träger enthält im Sinne der Erfindung mindestens 30 Gew.-%, insbesondere mindestens 50 Gew.-% Wasser, bezogen auf das Gesamtgewicht der Mittel. Erfindungsgemäß bevorzugt sind wässrige Träger.

Als ersten wesentlichen Inhaltsstoff (a) enthalten die erfindungsgemäßen Mittel mindestens ein kationisches Anthrachinonderivat der Formel (I) 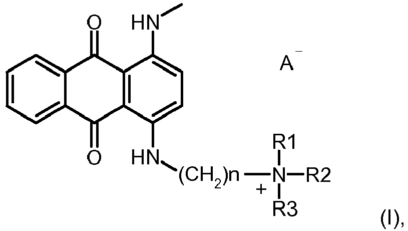 worin
- R1, R2, R3: unabhängig voneinander für eine C₁-C₆-Alkylgruppe stehen,
- n: für eine ganze Zahl von 2 bis 8 steht und
- A⁻: für ein physiologisch verträgliches Anion steht,

Hierbei stehen die Reste R1, R2 und R3 unabhängig voneinander für eine C₁-C₆-Alkylgruppe.
In einer bevorzugten Ausführungsform stehen R1 und R2 jeweils für eine Methylgruppe.
In einer weiteren bevorzugten Ausführungsform steht R3 für eine Methylgruppe, eine Ethylgruppe, eine n-Propylgruppe oder eine iso-Propylgruppe.
Ganz besonders bevorzugt stehen R1 und R2 jeweils für eine Methylgruppe, und R3 steht für eine n-Propylgruppe.

Weiterhin ist es bevorzugt, wenn n für die Zahlen 2 oder 3 steht, ganz besonders bevorzugt steht n für die Zahl 3.

A⁻ steht für ein physiologisch verträgliches Anion. Geeignete physiologisch verträgliche Anionen sind Halogenid, Hydrogensulfat, ½ Sulfat, Benzolsulfonat, p-Toluolsulfonat, Acetat, Citrat, Lactat, ½ Tartrat, Methylsulfat (H₃COSO₃⁻) oder Trifluormethansulfonat. Besonders bevorzugt steht A⁻ für Bromid oder für Methylsulfat (H₃COSO₃⁻), ganz besonders bevorzugt steht A⁻ für Bromid.

Eine besonders bevorzugte Ausführungsform ist ein Mittel zum Färben von keratinischen Fasern, welches dadurch gekennzeichnet ist, dass es mindestens ein kationisches Anthrachinonderivat (a) der Formel (I) enthält, bei dem
- R1 und R2 jeweils für eine Methylgruppe stehen,
- R3 für eine n-Propylgruppe steht und
- n für die Zahlen 2 oder 3, bevorzugt für die Zahl 3, steht.

Besonders intensive und glänzende Färbungen können erzeugt werden, wenn im erfindungsgemäßen Mittel als kationisches Anthrachinonderivat der Formel (I) die Verbindung (la) eingesetzt wird, 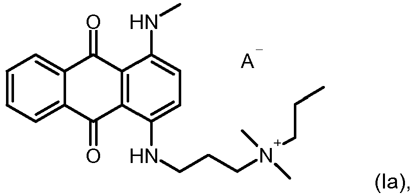 worin A⁻ für ein physiologisch verträgliches Anion, bevorzugt für Methylsulfat (H₃COSO₃⁻) oder Bromid, besonders bevorzugt für Bromid, steht.

Die Verbindung (la), bei der A⁻ für Bromid steht, ist auch unter der Bezeichnung HC Blue 16 oder Bluequat B, Bluequat Bromid oder unter dem chemischen Namen 3-{[9,10-Dihydro-4-(methyl-amino)-9,10-dioxo-1-anthracenyl]amino}-N,N-dimethyl-N-propyl-1-propanaminiumbromid bekannt.

Die erfindungsgemäßen Mittel zum Färben von Keratinfasern enthalten die Verbindung(en) der Formel (I) vorzugsweise in Mengen von größer gleich 0,0005 Gew.-% und von kleiner gleich 0,5 Gew.-%, jeweils bezogen auf das Gesamtgewicht des Mittels. Berechnungsgrundlage für diese Mengenangaben ist jeweils die Gesamtmenge aller im erfindungsgemäßen Mittel enthaltenden Anthrachinonfarbstoffe der Formel (I).

In einer besonders bevorzugten Ausführungsform ist ein erfindundungemäßes Mittel dadurch gekennzeichnet, dass es ein oder mehrere kationische Anthrachinonderivate der Formel (I) in einer Gesamtmenge von 0,0005 bis 0,5 Gew.-%, bevorzugt von 0,02 bis 0,4 Gew.-%, weiter bevorzugt von 0,05 bis 0,3 Gew.-% und insbesondere bevorzugt von 0,10 bis 0,35 Gew.-% -jeweils bezogen auf das Gesamtgewicht des Mittels - enthält.

Als zweiten wesentlichen Bestandteil enthält das erfindungsgemäße Mittel mindestens einen weiteren kationischen direktziehenden Farbstoff (b), der von Formel (I) verschieden ist. Unter einem kationischen Farbstoff wird hierbei ein Farbstoff mit einer kationischen Ladung verstanden.

Diese kationische Ladung kann sich hierbei an einem quartären Stickstoffatom befinden. Quartäre Stickstoffatome besitzen vier unterschiedliche organische Reste, aufgrund derer das Stickstoffatom positiv geladen ist. Es ist aber ebenso möglich, dass sich die kationische Ladung an einem stickstoffhaltigen Heterozyklus des direktziehenden Farbstoffes befindet. Wenn dieser Heterozyklus Teil des chromophoren Systems ist, kann die positive Ladung des direktziehenden Farbstoffs auch über das gesamte chromophore System delokalisiert sein. Schließlich ist es ebenfalls möglich, dass es sich bei dem kationischen Farbstoff um einen kationischen Triarylmethanfarbstoff handelt, bei dem die kationische Ladung innerhalb des Triarylmethan-Systems delokalisiert ist.

Besonders intensive Färbeergebnisse in Verbindung mit einem hohen und langanhaltenden Glanz können dann erzeugt werden, wenn das erfindungsgemäße Mittel als weiteren kationischen direktziehenden Farbstoff (b), der von Formel (I) verschieden ist, mindestens einen Farbstoff aus der Gruppe Basic Blue 7, Basic Blue 26, Basic Violet 2, Basic Violet 14, Basic Yellow 57, Basic Red 76, Basic Blue 99, Basic Brown 16, Basic Brown 17, Basic Yellow 87, Basic Orange 31, Basic Red 51 und Cationic Blue 347 enthält.

Eine weitere besonders bevorzugte Ausführungsform ist daher ein Mittel, welches dadurch gekennzeichnet ist, dass es als weiteren kationischen direktziehenden Farbstoff (b), der von Formel (I) verschieden ist, mindestens einen Farbstoff aus der Gruppe Basic Blue 7, Basic Blue 26, Basic Violet 2, Basic Violet 14, Basic Yellow 57, Basic Red 76, Basic Blue 99, Basic Brown 16, Basic Brown 17, Basic Yellow 87, Basic Orange 31, Basic Red 51 und Cationic Blue 347 enthält.

Ein weiterer bevorzugter Gegenstand der vorliegenden Erfindung ist ein Mittel zum Färben von keratinischen Fasern, enthaltend in einem kosmetischen Träger
(a) Bluequat B als kationisches Anthrachinonderivat der Formel (I),
(b) Basic Blue 7 als weiteren kationischen direktziehenden Farbstoff,
(c) mindestens ein amphoteres und/oder zwitterionisches Tensid und
(d) mindestens ein kationisches Polymer.

Ein weiterer bevorzugter Gegenstand der vorliegenden Erfindung ist ein Mittel zum Färben von keratinischen Fasern, enthaltend in einem kosmetischen Träger
(a) Bluequat B als kationisches Anthrachinonderivat der Formel (I),
(b) Basic Blue 26 als weiteren kationischen direktziehenden Farbstoff,
(c) mindestens ein amphoteres und/oder zwitterionisches Tensid und
(d) mindestens ein kationisches Polymer.

Ein weiterer bevorzugter Gegenstand der vorliegenden Erfindung ist ein Mittel zum Färben von keratinischen Fasern, enthaltend in einem kosmetischen Träger
(a) Bluequat B als kationisches Anthrachinonderivat der Formel (I),
(b) Basic Violet 2 als weiteren kationischen direktziehenden Farbstoff,
(c) mindestens ein amphoteres und/oder zwitterionisches Tensid und
(d) mindestens ein kationisches Polymer.

Ein weiterer bevorzugter Gegenstand der vorliegenden Erfindung ist ein Mittel zum Färben von keratinischen Fasern, enthaltend in einem kosmetischen Träger
(a) Bluequat B als kationisches Anthrachinonderivat der Formel (I),
(b) Basic Violet 14 als weiteren kationischen direktziehenden Farbstoff,
(c) mindestens ein amphoteres und/oder zwitterionisches Tensid und
(d) mindestens ein kationisches Polymer.

Ein weiterer bevorzugter Gegenstand der vorliegenden Erfindung ist ein Mittel zum Färben von keratinischen Fasern, enthaltend in einem kosmetischen Träger
(a) Bluequat B als kationisches Anthrachinonderivat der Formel (I),
(b) Basic Yellow 57 als weiteren kationischen direktziehenden Farbstoff,
(c) mindestens ein amphoteres und/oder zwitterionisches Tensid und
(d) mindestens ein kationisches Polymer.

Ein weiterer bevorzugter Gegenstand der vorliegenden Erfindung ist ein Mittel zum Färben von keratinischen Fasern, enthaltend in einem kosmetischen Träger
(a) Bluequat B als kationisches Anthrachinonderivat der Formel (I),
(b) Basic Red 76 als weiteren kationischen direktziehenden Farbstoff,
(c) mindestens ein amphoteres und/oder zwitterionisches Tensid und
(d) mindestens ein kationisches Polymer.

Ein weiterer bevorzugter Gegenstand der vorliegenden Erfindung ist ein Mittel zum Färben von keratinischen Fasern, enthaltend in einem kosmetischen Träger
(a) Bluequat B als kationisches Anthrachinonderivat der Formel (I),
(b) Basic Blue 99 als weiteren kationischen direktziehenden Farbstoff,
(c) mindestens ein amphoteres und/oder zwitterionisches Tensid und
(d) mindestens ein kationisches Polymer.

Ein weiterer bevorzugter Gegenstand der vorliegenden Erfindung ist ein Mittel zum Färben von keratinischen Fasern, enthaltend in einem kosmetischen Träger
(a) Bluequat B als kationisches Anthrachinonderivat der Formel (I),
(b) Basic Brown 16 als weiteren kationischen direktziehenden Farbstoff,
(c) mindestens ein amphoteres und/oder zwitterionisches Tensid und
(d) mindestens ein kationisches Polymer.

Ein weiterer bevorzugter Gegenstand der vorliegenden Erfindung ist ein Mittel zum Färben von keratinischen Fasern, enthaltend in einem kosmetischen Träger
(a) Bluequat B als kationisches Anthrachinonderivat der Formel (I),
(b) Basic Brown 17 als weiteren kationischen direktziehenden Farbstoff,
(c) mindestens ein amphoteres und/oder zwitterionisches Tensid und
(d) mindestens ein kationisches Polymer.

Ein weiterer bevorzugter Gegenstand der vorliegenden Erfindung ist ein Mittel zum Färben von keratinischen Fasern, enthaltend in einem kosmetischen Träger
(a) Bluequat B als kationisches Anthrachinonderivat der Formel (I),
(b) Basic Yellow 87 als weiteren kationischen direktziehenden Farbstoff,
(c) mindestens ein amphoteres und/oder zwitterionisches Tensid und
(d) mindestens ein kationisches Polymer.

Ein weiterer bevorzugter Gegenstand der vorliegenden Erfindung ist ein Mittel zum Färben von keratinischen Fasern, enthaltend in einem kosmetischen Träger
(a) Bluequat B als kationisches Anthrachinonderivat der Formel (I),
(b) Basic Orange 31 als weiteren kationischen direktziehenden Farbstoff,
(c) mindestens ein amphoteres und/oder zwitterionisches Tensid und
(d) mindestens ein kationisches Polymer.

Ein weiterer bevorzugter Gegenstand der vorliegenden Erfindung ist ein Mittel zum Färben von keratinischen Fasern, enthaltend in einem kosmetischen Träger
(a) Bluequat B als kationisches Anthrachinonderivat der Formel (I),
(b) Basic Red 51 als weiteren kationischen direktziehenden Farbstoff,
(c) mindestens ein amphoteres und/oder zwitterionisches Tensid und
(d) mindestens ein kationisches Polymer.

Ein weiterer bevorzugter Gegenstand der vorliegenden Erfindung ist ein Mittel zum Färben von keratinischen Fasern, enthaltend in einem kosmetischen Träger
(a) Bluequat B als kationisches Anthrachinonderivat der Formel (I),
(b) Cationic Blue 347 als weiteren kationischen direktziehenden Farbstoff,
(c) mindestens ein amphoteres und/oder zwitterionisches Tensid und
(d) mindestens ein kationisches Polymer.

Innerhalb der vorgenannten Gruppe der von Formel (I) verschiedenen kationischen direktziehenden Farbstoffe (b) zeichnen sich ganz bestimmte Farbstoffe dadurch aus, dass sich mit ihnen Färbungen von besonders herausragendem Glanz erzeugen lassen, wenn sie in Kombination mit den weiteren wesentlichen Bestandteilen (a), (c) und (d) auf keratinischen Fasern ausgefärbt werden. Ein besonders hoher Glanz kann erzielt werden, wenn als weiterer kationischer direktziehender Farbstoff (b) mindestens eine Verbindung aus der Gruppe Basic Yellow 87, Basic Orange 31, Basic Red 51, Basic Violet 2 und Cationic Blue 347 eingesetzt wird.

Der stärkste Glanz lässt sich mit einer oder mehrerer der Verbindung aus der Gruppe Basic Yellow 87, Basic Orange 31, Basic Red 51 Basic und Violet 2 in Kombination mit den weiteren wesentlichen Bestandteilen (a), (c) und (d) erhalten.

In einer explizit ganz besonders bevorzugten Ausführungsform ist ein erfindungsgemäßes Mittel deshalb dadurch gekennzeichnet, dass es sich bei dem oder bei den kationischen direktziehenden Farbstoffen (b), die von der Formel (I) verschieden sind, um mindestens einen Farbstoff ausgewählt aus der Gruppe Basic Yellow 87, Basic Orange 31, Basic Red 51, Basic Violet 2 und Cationic Blue 347, bevorzugt aus der Gruppe Basic Yellow 87, Basic Orange 31, Basic Red 51 und Basic Violet 2, handelt.

Bei den bevorzugten und besonders bevorzugten kationischen direktziehenden Farbstoffen (b) handelt es sich hierbei um die folgenden Verbindungen

| | |
|---|---|
| Basic Blue 7 | Basic Blue 26 |
| CAS-Nr. 2390-60-5 | CAS-Nr. 2580-56-5 |
| 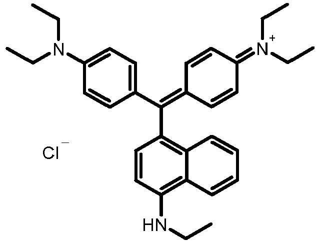 | 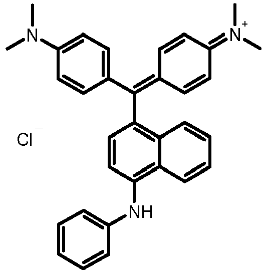 |
| Basic Violet 2 | Basic Violet 14 |
| CAS-Nr. 3248-91-7 | CAS-Nr. 632-99-5 |
| 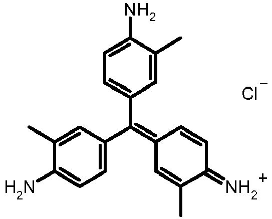 | 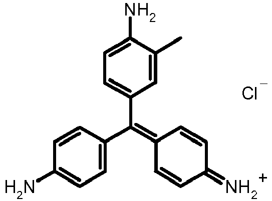 |
| Basic Yellow 57 | Basic Red 76 |
| CAS-Nr. 68391-31-1 | CAS-Nr. 68391-30-0 |
| 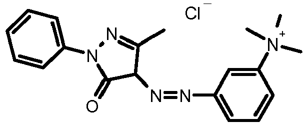 | 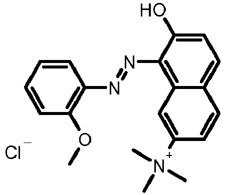 |
| Basic Blue 99 | Basic Brown 16 |
| CAS-Nr. 68123-13-7 | CAS-Nr. 26381-41-9 |
| 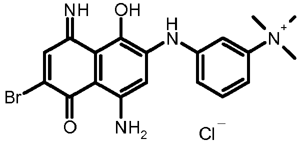 | 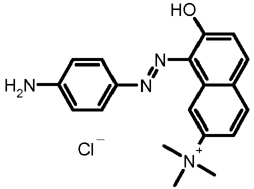 |
| Basic Brown 17 | Basic Yellow 87 |
| CAS-Nr. 176742-32-8 | CAS-N 68259-00-7 |
| 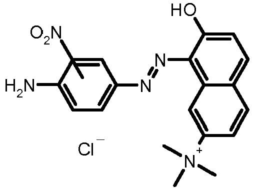 | 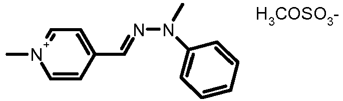 |
| Basic Orange 31 | Basic Red 51 |
| CAS-Nr. 97404-02-9 | CAS-Nr. 77061-58-6 |
| 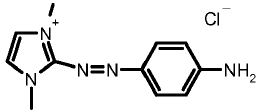 | 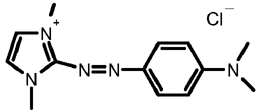 |
| Cationic Blue 347 | |
| CAS-Nr. 16517-75-2 | |
| 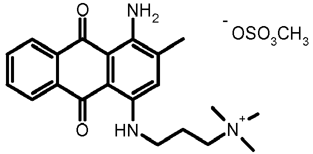 | |

Die kationischen direktziehenden Farbstoffe, die von Formel (I) verschieden sind, sind im erfindungsgemäßen Mittel in bevorzugten Mengenbereichen enthalten. Hierbei sollten die Mengen so gewählt sein, dass sie ausreichend hoch sind, um einen hohen Glanz zu erzielen, auf der anderen Seite jedoch niedrig genug sind, um die mit zu hohen Einatzkonzentrationen der Farbstoffen verbundene Nachteile wie erhöhte Hautanfärbungen oder mögliche Hautirritationen zu vermeiden.

Diese Voraussetzungen sind erfüllt, wenn die erfindungsgemäßen Mittel einen oder mehrere von Formel (I) verschiedene kationische direktziehende Farbstoffe (b) in einer Gesamtmenge von größer gleich 0,0005 und geringer gleich 0,55 Gew.-% - jeweils bezogen auf das Gesamtgewicht des Mittels - enthalten.

In einer weiteren besonders bevorzugten Ausführungsform ist ein erfindungsgemäßes Mittel dadurch gekennzeichnet, dass es ein oder mehrere kationische direktziehende Farbstoffe (b), die von Formel (I) verschieden sind, in einer Gesamtmenge von 0,0005 bis 0,55 Gew.-%, bevorzugt von 0,005 bis 0,40 Gew.-%, weiter bevorzugt von 0,025 bis 0,30 Gew.-% und insbesondere bevorzugt von 0,05 bis 0,20 Gew.-% - jeweils bezogen auf das Gesamtgewicht des Mittels - enthält.

Als dritten wesentlichen Bestandteil enthalten die erfindungsgemäßen Mittel mindestens ein amphoteres und/oder zwitterionisches Tensid (c).

Tenside sind amphiphile (bifunktionelle) Verbindungen, die aus mindestens einem hydrophoben und mindestens einem hydrophilen Molekülteil bestehen. Der hydrophobe Rest ist bevorzugt eine Kohlenwasserstoffkette mit 8-24 Kohlenstoff-Atomen, die gesättigt oder ungesättigt, linear oder verzweigt sein kann. Besonders bevorzugt ist diese C₈-C₂₄-Alkylkette linear.

Basiseigenschaften der Tenside sind die orientierte Absorption an Grenzflächen sowie die Aggragation zu Micellen und die Ausbildung von lyotropen Phasen.

Die amphoteren Tenside werden in ampholytische Tenside und zwitterionische Tenside unterteilt. Unter ampholytischen Tensiden werden solche oberflächenaktiven Verbindungen verstanden, die sowohl saure (beispielsweise -COOH oder -SO₃H-Gruppen) als auch basische hydrophile Gruppen (beispielsweise Aminogruppen) besitzen und sich also je nach Bedingung sauer oder basisch verhalten. Unter zwitterionischen Tensiden versteht der Fachmann Tenside, die im selben Molekül sowohl eine negative als auch eine positive Ladung tragen.

Beispiele für bevorzugte zwitterionische Tenide (c) sind die Betaine, die N-Alkyl-N,N-dimethyl-ammonium-glycinate, die N-Acyl-aminopropyl-N,N-dimethylammoniumglycinate und die 2-Alkyl-3-carboxymethyl-3-hydroxyethyl-imidazoline mit jeweils 8 bis 24-Catomen in der Alkylgruppe

Beispiele für bevorzugte ampholytische Tenside (c) sind N-Alkylglycine, N-Alkylaminopropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils 8 bis 24 C-Atomen in der Alkylgruppe.

In einer weiteren bevorzugten Ausführungsform ist ein erfindungsgemäßes Mittel deshalb dadurch gekennzeichnet, dass es als amphoteres und/oder zwitterionisches Tensid (c) mindestens ein Tensid aus der Gruppe der Betaine, der N-Alkyl-N,N-dimethylammonium-glycinate, der N-Acylaminopropyl-N,N-dimethylammoniumglycinate, der 2-Alkyl-3-carboxymethyl-3-hydroxyethylimidazoline, der N-Alkylglycine, der N-Alkylpropionsäuren, der N-Alkylaminobuttersäuren, der N-Alkyliminodipropionsäuren, der N-Hydroxyethyl-N-alkylamidopropylglycine, der N-Alkyltaurine, der N-Alkylsarcosine, der 2-Alkylaminopropionsäuren und der Alkylaminoessigsäuren enthält. Weiterhin bevorzugte amphotere und/oder zwitterionische Tenside (c) sind die Tenside der nachfolgenden Formeln (II) bis (XIII).

Ein weiterer bevorzugter Gegenstand der vorliegenden Erfindung ist ein Mittel zum Färben von keratinischen Fasern, enthaltend in einem kosmetischen Träger
(a) mindestens ein kationisches Anthrachinonderivat der Formel (I), worin R1, R2, R3, n und A⁻ wie zuvor beschrieben definiert sind,
(b) mindestens einen weiteren kationischen direktziehenden Farbstoff, der von Formel (I) verschieden ist,
(c) mindestens ein amphoteres und/oder zwitterionisches Tensid der Formel (II) 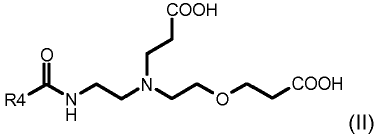 worin R4 für eine gesättigte oder ungesättigte, verzweigte oder unverzweigte C₈-C₂₄-Alkylgruppe steht und
(d) mindestens ein kationisches Polymer.

Ein weiterer bevorzugter Gegenstand der vorliegenden Erfindung ist ein Mittel zum Färben von keratinischen Fasern, enthaltend in einem kosmetischen Träger
(a) mindestens ein kationisches Anthrachinonderivat der Formel (I), worin R1, R2, R3, n und A⁻ wie zuvor beschrieben definiert sind,
(b) mindestens einen weiteren kationischen direktziehenden Farbstoff, der von Formel (I) verschieden ist,
(c) mindestens ein amphoteres und/oder zwitterionisches Tensid der Formel (III) 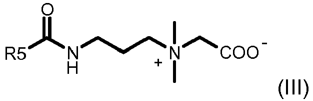 worin R5 für eine gesättigte oder ungesättigte, verzweigte oder unverzweigte C₈-C₂₄-Alkylgruppe steht und
(d) mindestens ein kationisches Polymer.

Ein weiterer bevorzugter Gegenstand der vorliegenden Erfindung ist ein Mittel zum Färben von keratinischen Fasern, enthaltend in einem kosmetischen Träger
(a) mindestens ein kationisches Anthrachinonderivat der Formel (I), worin R1, R2, R3, n und A⁻ wie zuvor beschrieben definiert sind,
(b) mindestens einen weiteren kationischen direktziehenden Farbstoff, der von Formel (I) verschieden ist,
(c) mindestens ein amphoteres und/oder zwitterionisches Tensid der Formel (IV) 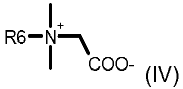 worin R6 für eine gesättigte oder ungesättigte, verzweigte oder unverzweigte C₈-C₂₄-Alkylgruppe steht und
(d) mindestens ein kationisches Polymer.

Ein weiterer bevorzugter Gegenstand der vorliegenden Erfindung ist ein Mittel zum Färben von keratinischen Fasern, enthaltend in einem kosmetischen Träger
(a) mindestens ein kationisches Anthrachinonderivat der Formel (I), worin R1, R2, R3, n und A⁻ wie zuvor beschrieben definiert sind,
(b) mindestens einen weiteren kationischen direktziehenden Farbstoff, der von Formel (I) verschieden ist,
(c) mindestens ein amphoteres und/oder zwitterionisches Tensid der Formel (V) 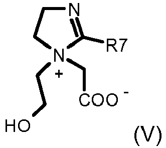 worin R7 für eine gesättigte oder ungesättigte, verzweigte oder unverzweigte C₈-C₂₄-Alkylgruppe steht und
(d) mindestens ein kationisches Polymer.

Ein weiterer bevorzugter Gegenstand der vorliegenden Erfindung ist ein Mittel zum Färben von keratinischen Fasern, enthaltend in einem kosmetischen Träger
(a) mindestens ein kationisches Anthrachinonderivat der Formel (I), worin R1, R2, R3, n und A⁻ wie zuvor beschrieben definiert sind,
(b) mindestens einen weiteren kationischen direktziehenden Farbstoff, der von Formel (I) verschieden ist,
(c) mindestens ein amphoteres und/oder zwitterionisches Tensid der Formel (VI) 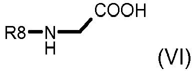 worin R8 für eine gesättigte oder ungesättigte, verzweigte oder unverzweigte C₈-C₂₄-Alkylgruppe steht und
(d) mindestens ein kationisches Polymer.

Ein weiterer bevorzugter Gegenstand der vorliegenden Erfindung ist ein Mittel zum Färben von keratinischen Fasern, enthaltend in einem kosmetischen Träger
(a) mindestens ein kationisches Anthrachinonderivat der Formel (I), worin R1, R2, R3, n und A⁻ wie zuvor beschrieben definiert sind,
(b) mindestens einen weiteren kationischen direktziehenden Farbstoff, der von Formel (I) verschieden ist,
(c) mindestens ein amphoteres und/oder zwitterionisches Tensid der Formel (VII) 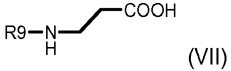 worin R9 für eine gesättigte oder ungesättigte, verzweigte oder unverzweigte C₈-C₂₄-Alkylgruppe steht und
(d) mindestens ein kationisches Polymer.

Ein weiterer bevorzugter Gegenstand der vorliegenden Erfindung ist ein Mittel zum Färben von keratinischen Fasern, enthaltend in einem kosmetischen Träger
(a) mindestens ein kationisches Anthrachinonderivat der Formel (I), worin R1, R2, R3, n und A⁻ wie zuvor beschrieben definiert sind,
(b) mindestens einen weiteren kationischen direktziehenden Farbstoff, der von Formel (I) verschieden ist,
(c) mindestens ein amphoteres und/oder zwitterionisches Tensid der Formel (VIII) 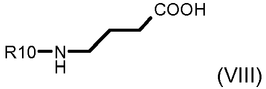 worin R10 für eine gesättigte oder ungesättigte, verzweigte oder unverzweigte C₈-C₂₄-Alkylgruppe steht und
(d) mindestens ein kationisches Polymer.

Ein weiterer bevorzugter Gegenstand der vorliegenden Erfindung ist ein Mittel zum Färben von keratinischen Fasern, enthaltend in einem kosmetischen Träger
(a) mindestens ein kationisches Anthrachinonderivat der Formel (I), worin R1, R2, R3, n und A⁻ wie zuvor beschrieben definiert sind,
(b) mindestens einen weiteren kationischen direktziehenden Farbstoff, der von Formel (I) verschieden ist,
(c) mindestens ein amphoteres und/oder zwitterionisches Tensid der Formel (IX) 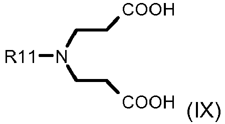 worin R11 für eine gesättigte oder ungesättigte, verzweigte oder unverzweigte C₈-C₂₄-Alkylgruppe steht und
(d) mindestens ein kationisches Polymer.

Ein weiterer bevorzugter Gegenstand der vorliegenden Erfindung ist ein Mittel zum Färben von keratinischen Fasern, enthaltend in einem kosmetischen Träger
(a) mindestens ein kationisches Anthrachinonderivat der Formel (I), worin R1, R2, R3, n und A⁻ wie zuvor beschrieben definiert sind,
(b) mindestens einen weiteren kationischen direktziehenden Farbstoff, der von Formel (I) verschieden ist,
(c) mindestens ein amphoteres und/oder zwitterionisches Tensid der Formel (X) 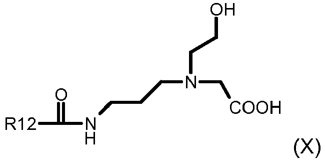 worin R12 für eine gesättigte oder ungesättigte, verzweigte oder unverzweigte C₈-C₂₄-Alkylgruppe steht und
(d) mindestens ein kationisches Polymer.

Ein weiterer bevorzugter Gegenstand der vorliegenden Erfindung ist ein Mittel zum Färben von keratinischen Fasern, enthaltend in einem kosmetischen Träger
(a) mindestens ein kationisches Anthrachinonderivat der Formel (I), worin R1, R2, R3, n und A⁻ wie zuvor beschrieben definiert sind,
(b) mindestens einen weiteren kationischen direktziehenden Farbstoff, der von Formel (I) verschieden ist,
(c) mindestens ein amphoteres und/oder zwitterionisches Tensid der Formel (XI) 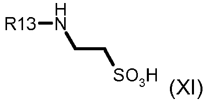 worin R13 für eine gesättigte oder ungesättigte, verzweigte oder unverzweigte C₈-C₂₄-Alkylgruppe steht und
(d) mindestens ein kationisches Polymer.

Ein weiterer bevorzugter Gegenstand der vorliegenden Erfindung ist ein Mittel zum Färben von keratinischen Fasern, enthaltend in einem kosmetischen Träger
(a) mindestens ein kationisches Anthrachinonderivat der Formel (I), worin R1, R2, R3, n und A⁻ wie zuvor beschrieben definiert sind,
(b) mindestens einen weiteren kationischen direktziehenden Farbstoff, der von Formel (I) verschieden ist,
(c) mindestens ein amphoteres und/oder zwitterionisches Tensid der Formel (XII) 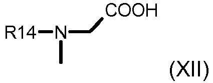 worin R14 für eine gesättigte oder ungesättigte, verzweigte oder unverzweigte C₈-C₂₄-Alkylgruppe steht und
(d) mindestens ein kationisches Polymer.

Ein weiterer bevorzugter Gegenstand der vorliegenden Erfindung ist ein Mittel zum Färben von keratinischen Fasern, enthaltend in einem kosmetischen Träger
(a) mindestens ein kationisches Anthrachinonderivat der Formel (I), worin R1, R2, R3, n und A⁻ wie zuvor beschrieben definiert sind,
(b) mindestens einen weiteren kationischen direktziehenden Farbstoff, der von Formel (I) verschieden ist,
(c) mindestens ein amphoteres und/oder zwitterionisches Tensid der Formel (XIII) 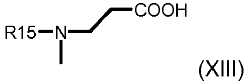 worin R15 für eine gesättigte oder ungesättigte, verzweigte oder unverzweigte C₈-C₂₄-Alkylgruppe steht und
(d) mindestens ein kationisches Polymer.

Innerhalb der Gruppe der amphoteren und/oder zwitterionischen Tenside sind die Verbindungen der Formeln (II) und/oder (III) ganz besonders bevorzugt, da bei Einsatz dieser Verbindungen der stärkste Glanz auf keratinischen Fasern erzeugt werden kann.

In einer weiteren ganz besonders bevorzugten Ausführungsform ist ein erfindungsgemäßes Mittel deshalb dadurch gekennzeichnet, dass es als amphoteres und/oder zwitterionisches Tensid (c) mindestens eine Verbindung der Formel (II) und/oder der Formel (III) enthält, 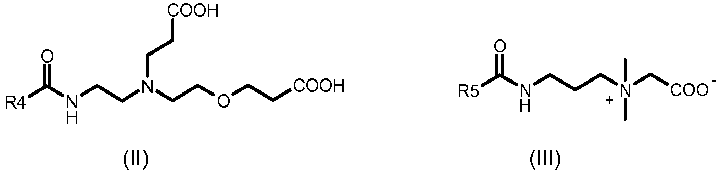 worin
R4, R5 jeweils unabhängig voneinander für eine gesättigte oder ungesättigte, verzweigte oder unverzweigte C₈-C₂₄-Alkylgruppe stehen.

Die Verbindungen der Strukturen (II) bis (X) sowie (XII) und (XIII) enthalten Carbonsäuregruppierungen. In wässrigem Medium spalten Carbonsäuregruppierungen abhängig vom pH-Wert des sie umgebenden Mediums ein Proton ab und liegen dann im Gleichgewicht mit ihrem jeweiligen Carboxylat-Anion vor. Neben dem jeweiligen Tensid in der protonierten Form (-COOH) ist auch seine deprotonierte Form (-COO-) erfindungsgemäß. Zur Wahrung der Elektroneutralität kann die deprotonierte Carbonsäure auch in Form ihres jeweiligen Salzes, vorzugsweise ihres Alkalimetallsalzes (Na, K) vorliegen.

Zur Lösung der erfindungsgemäßen Aufgabenstellung ganz besonders geeignete amphotere und/oder zwitterionische Tenside der Formel (II) sind die unter der INCI-Bezeichnung Disodium Cocoamphodipropionate bekannten Verbindungen.

Zur Lösung der erfindungsgemäßen Aufgabenstellung ganz besonders geeignete amphotere und/oder zwitterionische Tenside der Formel (III) sind die unter der INCI-Bezeichnung Cocamidopropyl Betain bekannten Verbindungen.

Die amphoteren und/oder zwitterionischen Tenside (c) sind in den erfindungsgemäßen Mitteln bevorzugt in bestimmten Mengenbereichen enthalten. Berechnungsgundlage für die nachfolgend genannten Mengenangabe ist die Gesamtmenge aller im jeweiligen Mittel enthaltenen amphoteren Tenside und zwitterionischen Tenside, wobei die Mengen jeweils auf das Gesamtgewicht des Mittels bezogen sind.

In einer weiteren bevorzugten Ausführungsform ist ein erfindungsgemäßes Mittel dadurch gekennzeichnet, dass es ein oder mehrere amphotere und/oder zwitterionische Tenside (c) in einer Gesamtmenge von 0,1 bis 4,0 Gew.-%, bevorzugt von 0,2 bis 2,5 Gew.-%, weiter bevorzugt von 0,4 bis 1,8 Gew.-% und insbesondere bevorzugt von 0,6 bis 0,9 Gew.-% -jeweils bezogen auf das Gesamtgewicht des Mittels - enthält.

Als vierten wesentlichen Bestandteil enthalten die erfindungsgemäßen Mittel mindestens ein kationisches Polymer (d).

Unter Polymeren werden Makromoleküle mit einem Molekulargewicht von mindestens 1000 g/mol, bevorzugt von mindestens 2500 g/mol, besonders bevorzugt von mindestens 5000 g/mol, verstanden, welche aus gleichen, sich wiederholenden organischen Einheiten bestehen. Polymere werden durch Polymerisation eines Monomertyps oder durch Polymerisation von verschiedenen, strukturell voneinander unterschiedlichen Monomertypen hergestellt. Wird das Polymer durch Polymerisation eines Monomertyps hergestellt, spricht man von Homo-Polymeren. Werden strukturell unterschiedliche Monomertypen in der Polymerisation eingesetzt, wird das resultiertende Polymer als Copolymer bezeichnet.

Das maximale Molekulargewicht des Polymers hängt vom Polymerisationsgrad (Anzahl der polymerisierten Monomere) ab und wird durch die Polymerisationsmethode mitbestimmt. Im Sinne der vorliegenden Erfindung ist es bevorzugt, wenn das maximale Molekulargewicht des kationischen Polymers (d) nicht mehr als 10⁷ g/mol, bevorzugt nicht mehr als 10⁶ g/mol und besonders bevorzugt nicht mehr als 10⁵ g/mol beträgt.

Unter kationischen Polymeren werden solche Polymere verstanden, die im Makromolekül (Polymer) kationische Gruppierungen enthalten. Bei diesen im Makromolekül (Polymer) enthaltenen kationischen Gruppierungen handelt es sich quartäre Ammoniumgruppen.

Im Zuge der zu dieser Erfindung führenden Arbeiten hat sich gezeigt, dass insbesondere dann besonders intensive Färbungen und hohe Glanzwerte erzielt werden konnten, wenn spezielle kationische Polymere zusammen mit den weiteren wesentlichen Bestandteilen (a), (b) und (c) in einer Formulierung zum Einsatz kommen.

Besonders hoher und lang anhaltender Glanz konnte erzielt werden, wenn als kationisches Polymer (d) mindestens ein Polymer aus der Gruppe Polyquaternium-2, Polyquaternium-5, Polyquaternium-6, Polyquaternium-7, Polyquaternium-8, Polyquaternium-9, Polyquaternium-11, Polyquaternium-16, Polyquaternium-28, Polyquaternium-32, Polyquaternium-33, Polyquaternium-44, Polyquaternium-46, Polyquatenrium-55 und Polyquaternium-68 im erfindungsgemäßen Mittel enthalten ist.

In einer weiteren besonders bevorzugten Ausführungsform ist ein erfindungsgemäßes Mittel deshalb dadurch gekennzeichnet, dass es als kationisches Polymer (d) mindestens ein Polymer aus der Gruppe Polyquaternium-2, Polyquaternium-5, Polyquaternium-6, Polyquaternium-7, Polyquaternium-8, Polyquaternium-9, Polyquaternium-11, Polyquaternium-16, Polyquaternium-28, Polyquaternium-32, Polyquaternium-33, Polyquaternium-44, Polyquaternium-46, Polyquaternium-55 und Polyquaternium-68 enthält.

Bei Polyquaternium-2 handelt es sich um Poly[oxy-1,2-ethanediyl(dimethyliminio)-1,3-propanediyliminocarbonylimino-1,3-propanediyl(dimethyliminio)-1,2-ethanediyl chloride (1:2), das beispielsweise unter den Handelsnamen Lugalvan P oder Mirapol A 15 vertrieben wird.

Polyquaternium-5 ist ein Copolymer aus *N*,*N*,*N*-Trimethyl-2-[(2-methyl-1-oxo-2-propen-1-yl)oxy]-ethanaminium-methylsulfat und 2-Propenamid und wird beispielsweise unter den Handelsnamen Calgon K 400, Catamer Q, Hercofloc 812 oder Merquat 5 vertrieben.

Polyquaternium-6 ist das Homopolymer aus N,N-Dimethyl-N-(2-propen-1-yl)-2-propen-1-aminiumchlorid und ist zum Beispiel unter den Handelsnamen Merquat 100 oder Genamin PDAC erhältlich.

Polyquaternium-7 ist das Copolymer aus N,N-Dimethyl-N-(2-propen-1-yl)-2-propen-1-aminiumchlorid und 2-Propenamid und wird beispielsweise unter den Handelsnamen Merquat 550, Merquat 2220 oder Rheocare CC 7 vertrieben.

Bei Polyquaternium-8 handelt es sich um das Copolymer aus 2-Methyl-2-propensäuremethylester, 2-(Dimethylamino)ethyl-2-methyl-2-propenoat und Octadecyl-2-methyl-2-propenoat, welches mit Dimethylsulfat quaterniert wurde.

Polyquaternium-9 ist das Homopolymer aus 2-(Dimethylamino)ethyl-2-methyl-2-propenoat, welches mit Methylbromid quaterniert wurde.

Bei Polyquaternium-11 hadelt es sich um das Copolymer aus 2-Methyl-2-propensäure-2-(dimethylamino)ethylester und 1-Ethenyl-2-pyrrolidinone, welches mit Diethylsulfat quaterniert wurde. Polyquaternium-11 wird beispielsweise unter den Handelsnamen Celquat 200, Gafquat 755 oder Luviquat PQ 11 vertrieben.

Polyquaternium-16 ist das Copolymer aus 1-Ethenyl-2-pyrrolidinon und 1-Ethenyl-3-methyl-1 H-imidazoliumchlorid und beispielsweise unter den Handelsnamen Luviquat FC 500 , Luviquat FC 550 oder Luviquat HM 552 bekannt.

Bei Polyquaternium-28 handelt es sich um ein Copolymer aus *N*,*N*,*N*-Trimethyl-3-[(2-methyl-1-oxo-2-propen-1-yl)amino]-1-propanaminiumchlorid und 1-Ethenyl-2-pyrrolidinon, beispielsweise unter den Handelsnamen Gafquat HS 100 oder Conditioneze NF 20 erhältlich.

Polyquaternium-32 ist ein Copolymer aus 2-Propenamid und N,N,N-Trimethyl-2-[(2-methyl-1-oxo-2-propen-1-yl)oxy]ethanaminiumchlorid und wird beispielsweise unter den Handelsnamen Salcare SC 92 oder Rohagit KF 720F vertrieben.

Bei Polyquaternium-33 handelt es sich um ein Copolymer aus 2-Propenamid und N,N,N-Trimethyl-2-[(1-oxo-2-propen-1-yl)oxy]ethanaminiumchlorid, das zum Beispiel unter den Handelsnamen Nalco 1460, Organopol 6400 oder Salcare SC 93 bekannt ist.

Polyquaternium-44 ist ein Copolymer aus 1-Ethenyl-3-methyl-1 H-imidazolium methylsulfat und 1-Ethenyl-2-pyrrolidinon und beispielsweise bekannt unter den Handelsnamen Luviquat Care oder Luviquat MS 370.

Bei Polyquaternium-46 handelt es sich um ein Copolymer aus 1-Ethenyl-2-pyrrolidinon, 1-Ethenyl-3-methyl-1 H-imidaolium methylsulfat und 1-Ethenylhexahydro-2*H*-azepin-2-on.

Polyquaternium-55 ist ein Copolymer aus 1-Ethenyl-2-pyrrolidinon, *N*-[3-(Dimethylamino)propyl]-2-methyl-2-propenamid und *N*,*N*-dimethyl-*N*-[3-[(2-methyl-1-oxo-2-propen-1-yl)amino]propyl]-1-dodecanaminiumchlorid und wird zum Beispiel unter den Handelsnamen Styleze W 10 oder Styleze W 20 vertrieben.

Bei Polyquaternium-68 handelt es sich schließlich um ein Copolymer aus 1-Ethenyl-3-methyl-1 H-imidazolium methylsulfat, 1-Ethenyl-1 H-imidazol, 1-Ethenyl-2-pyrrolidinon und 2-Methyl-2-propenamid.

Zur optimalen Lösung der erfindungsgemäßen Aufgabenstellung enthalten die erfindungsgemäßen Mittel auch die kationischen Polymere (d) in bevorzugten Mengenbereichen.

In einer weiteren bevorzugten Ausführungsform ist ein erfindungsgemäßes Mittel daher dadurch gekennzeichnet, dass es ein oder mehrere kationische Polymere (d) in einer Gesamtmenge von 0,1 bis 2,0 Gew.-%, bevorzugt von 0,2 bis 1,3 Gew.-%, weiter bevorzugt von 0,3 bis 0,9 Gew.-% und insbesondere bevorzugt von 0,4 bis 0,8 Gew.-% - jeweils bezogen auf das Gesamtgewicht des Mittels - enthält.

Die mit Abstand besten Glanzwerte wurden erhalten, wenn als kationisches Polymer (d) Polyquaternium-6 zusammen mit den weiteren wesentlichen Bestandteilen (a), (b) und (c) im erfindungsgemäßen Mittel eingesetzt wurde.

In einer weiteren explizit ganz besonders bevorzugten Ausführungsform ist ein erfindungsgemäßes Mittel daher dadurch gekennzeichnet, dass es als kationisches Polymer (d) Polyquaternium-6 in einer Menge von 0,0001 bis 10 Gew.-%, bevorzugt von 0,001 bis 8 Gew.-%, weiter bevorzugt von 0,01 bis 5 Gew.-% und insbesondere bevorzugt von 0,05 bis 2,5 Gew.-% - jeweils bezogen auf das Gesamtgewicht des Mittels - enthält.

Ein weiterer bevorzugter Gegenstand der vorliegenden Erfindung ist ein Mittel zum Färben von keratinischen Fasern, enthaltend in einem kosmetischen Träger
(a) Bluequat B als kationisches Anthrachinonderivat der Formel (I),
(b) Basic Blue 7 als weiteren kationischen direktziehenden Farbstoff,
(c) mindestens ein amphoteres und/oder zwitterionisches Tensid und
(d) als kationisches Polymer Polyquaternium-6 enthält.

Ein weiterer bevorzugter Gegenstand der vorliegenden Erfindung ist ein Mittel zum Färben von keratinischen Fasern, enthaltend in einem kosmetischen Träger
(a) Bluequat B als kationisches Anthrachinonderivat der Formel (I),
(b) Basic Blue 7 als weiteren kationischen direktziehenden Farbstoff,
(c) mindestens ein amphoteres und/oder zwitterionisches Tensid und
(d) als kationisches Polymer Polyquaternium-6 enthält.

Ein weiterer bevorzugter Gegenstand der vorliegenden Erfindung ist ein Mittel zum Färben von keratinischen Fasern, enthaltend in einem kosmetischen Träger
(a) Bluequat B als kationisches Anthrachinonderivat der Formel (I),
(b) Basic Blue 26 als weiteren kationischen direktziehenden Farbstoff,
(c) mindestens ein amphoteres und/oder zwitterionisches Tensid und
(d) als kationisches Polymer Polyquaternium-6 enthält.

Ein weiterer bevorzugter Gegenstand der vorliegenden Erfindung ist ein Mittel zum Färben von keratinischen Fasern, enthaltend in einem kosmetischen Träger
(a) Bluequat B als kationisches Anthrachinonderivat der Formel (I),
(b) Basic Violet 2 als weiteren kationischen direktziehenden Farbstoff,
(c) mindestens ein amphoteres und/oder zwitterionisches Tensid und
(d) als kationisches Polymer Polyquaternium-6 enthält.

Ein weiterer bevorzugter Gegenstand der vorliegenden Erfindung ist ein Mittel zum Färben von keratinischen Fasern, enthaltend in einem kosmetischen Träger
(a) Bluequat B als kationisches Anthrachinonderivat der Formel (I),
(b) Basic Violet 14 als weiteren kationischen direktziehenden Farbstoff,
(c) mindestens ein amphoteres und/oder zwitterionisches Tensid und
(d) als kationisches Polymer Polyquaternium-6 enthält.

Ein weiterer bevorzugter Gegenstand der vorliegenden Erfindung ist ein Mittel zum Färben von keratinischen Fasern, enthaltend in einem kosmetischen Träger
(a) Bluequat B als kationisches Anthrachinonderivat der Formel (I),
(b) Basic Yellow 57 als weiteren kationischen direktziehenden Farbstoff,
(c) mindestens ein amphoteres und/oder zwitterionisches Tensid und
(d) als kationisches Polymer Polyquaternium-6 enthält.

Ein weiterer bevorzugter Gegenstand der vorliegenden Erfindung ist ein Mittel zum Färben von keratinischen Fasern, enthaltend in einem kosmetischen Träger
(a) Bluequat B als kationisches Anthrachinonderivat der Formel (I),
(b) Basic Red 76 als weiteren kationischen direktziehenden Farbstoff,
(c) mindestens ein amphoteres und/oder zwitterionisches Tensid und
(d) als kationisches Polymer Polyquaternium-6 enthält.

Ein weiterer bevorzugter Gegenstand der vorliegenden Erfindung ist ein Mittel zum Färben von keratinischen Fasern, enthaltend in einem kosmetischen Träger
(a) Bluequat B als kationisches Anthrachinonderivat der Formel (I),
(b) Basic Blue 99 als weiteren kationischen direktziehenden Farbstoff,
(c) mindestens ein amphoteres und/oder zwitterionisches Tensid und
(d) als kationisches Polymer Polyquaternium-6 enthält.

Ein weiterer bevorzugter Gegenstand der vorliegenden Erfindung ist ein Mittel zum Färben von keratinischen Fasern, enthaltend in einem kosmetischen Träger
(a) Bluequat B als kationisches Anthrachinonderivat der Formel (I),
(b) Basic Brown 16 als weiteren kationischen direktziehenden Farbstoff,
(c) mindestens ein amphoteres und/oder zwitterionisches Tensid und
(d) als kationisches Polymer Polyquaternium-6 enthält.

Ein weiterer bevorzugter Gegenstand der vorliegenden Erfindung ist ein Mittel zum Färben von keratinischen Fasern, enthaltend in einem kosmetischen Träger
(a) Bluequat B als kationisches Anthrachinonderivat der Formel (I),
(b) Basic Brown 17 als weiteren kationischen direktziehenden Farbstoff,
(c) mindestens ein amphoteres und/oder zwitterionisches Tensid und
(d) als kationisches Polymer Polyquaternium-6 enthält.

Ein weiterer bevorzugter Gegenstand der vorliegenden Erfindung ist ein Mittel zum Färben von keratinischen Fasern, enthaltend in einem kosmetischen Träger
(a) Bluequat B als kationisches Anthrachinonderivat der Formel (I),
(b) Basic Yellow 87als weiteren kationischen direktziehenden Farbstoff,
(c) mindestens ein amphoteres und/oder zwitterionisches Tensid und
(d) als kationisches Polymer Polyquaternium-6 enthält.

Ein weiterer bevorzugter Gegenstand der vorliegenden Erfindung ist ein Mittel zum Färben von keratinischen Fasern, enthaltend in einem kosmetischen Träger
(a) Bluequat B als kationisches Anthrachinonderivat der Formel (I),
(b) Basic Orange 31 als weiteren kationischen direktziehenden Farbstoff,
(c) mindestens ein amphoteres und/oder zwitterionisches Tensid und
(d) als kationisches Polymer Polyquaternium-6 enthält.

Ein weiterer bevorzugter Gegenstand der vorliegenden Erfindung ist ein Mittel zum Färben von keratinischen Fasern, enthaltend in einem kosmetischen Träger
(a) Bluequat B als kationisches Anthrachinonderivat der Formel (I),
(b) Basic Red 51 als weiteren kationischen direktziehenden Farbstoff,
(c) mindestens ein amphoteres und/oder zwitterionisches Tensid und
(d) als kationisches Polymer Polyquaternium-6 enthält.

Ein weiterer bevorzugter Gegenstand der vorliegenden Erfindung ist ein Mittel zum Färben von keratinischen Fasern, enthaltend in einem kosmetischen Träger
(a) Bluequat B als kationisches Anthrachinonderivat der Formel (I),
(b) Cationic Blue 347 als weiteren kationischen direktziehenden Farbstoff,
(c) mindestens ein amphoteres und/oder zwitterionisches Tensid und
(d) als kationisches Polymer Polyquaternium-6 enthält.

Zusätzlich zu den kationischen Anthrachinonfarbstoffen der Formel (I) und den weiteren, von Formel (I) verschiedenen kationischen Farbstoffen (b) können die erfindungsgemäßen Mittel als optionale Bestandteile auch noch weitere nichtionische und/oder anionische direktziehende Farbstoffe enthalten.

Bevorzugte anionische direktziehende Farbstoffe sind die unter den internationalen Bezeichnungen bzw. Handelsnamen Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1, Acid Black 52, Bromphenolblau und Tetrabromphenolblau bekannten Verbindungen.

Als nichtionische direktziehende Farbstoffe eignen sich insbesondere nichtionische Nitro- und Chinonfarbstoffe und neutrale Azofarbstoffe. Bevorzugte nichtionische direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 7,HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9 bekannten Verbindungen, sowie 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)amino-5-chlor-2-nitrobenzol, 4-Amino-3-nitrophenol, 1-(2'-Ureidoethyl)amino-4-nitrobenzol, 2-[(4-Amino-2-nitrophenyl)amino]benzoesäure, 4-[(3-Hydroxypropyl)amino]-3-nitrophenol, 4-Nitro-o-phenylendiamin, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol.

Die erfindungsgemäßen Mittel können weiterhin auch als Oxidationsfärbemittel eingesetzt werden. Solche Oxidationsfärbemittel enthalten zusätzlich mindestens ein Oxidationsfarbstoffvorprodukt, bevorzugt zumindest ein Oxidationsfarbstoffvorprodukt vom Entwickler-Typ und mindestens ein Oxidationsfarbstoffvorprodukt vom Kuppler-Typ. Besonders geeignete Oxidationsfarbstoffvorprodukte vom Entwickler-Typ sind dabei ausgewählt aus mindestens einer Verbindung aus der Gruppe, die gebildet wird aus p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, 2-Methoxymethyl-p-phenylendiamin, N-(4-Amino-3-methylphenyl)-N-[3-(1H-imidazol-1-yl)-propyl]amin, N,N'-Bis-(2-hydroxyethyl)-N,N'-bis-(4-aminophenyl)-1,3-diamino-propan-2-ol, Bis-(2-hydroxy-5-aminophenyl)methan, 1,3-Bis-(2,5-diaminophenoxy)propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-Amino-3-methylphenol, 4-Amino-2-aminomethylphenol, 4-Amino-2-(1,2-dihydroxyethyl)-phenol, 4-Amino-2-(diethylaminomethyl)phenol, 4,5-Diamino-1-(2-hydroxyethyl)pyrazol, 2,4,5,6-Tetraaminopyrimidin, 4-Hydroxy-2,5,6-triaminopyrimidin, 2-Hydroxy-4,5,6-triaminopyrimidin, 2,3-Diamino-6,7-dihydro-1H,5H-pyrazolo[1,2-a]pyrazol-1-on sowie deren physiologisch verträglichen Salzen.

Besonders geeignete Oxidationsfarbstoffvorprodukte vom Kuppler-Typ sind dabei ausgewählt aus der Gruppe, gebildet aus 3-Aminophenol, 5-Amino-2-methylphenol, 3-Amino-2-chlor-6-methylphenol, 2-Hydroxy-4-aminophenoxyethanol, 5-Amino-4-chlor-2-methylphenol, 5-(2-Hydroxyethyl)-amino-2-methylphenol, 2,4-Dichlor-3-aminophenol, 2-Aminophenol, 3-Phenylendiamin, 2-(2,4-Diaminophenoxy)ethanol, 1,3-Bis(2,4-diaminophenoxy)propan, 1-Methoxy-2-amino-4-(2-hydroxyethylamino)benzol, 1,3-Bis(2,4-diaminophenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2-hydroxyethyl)aminobenzol, Resorcin, 2-Methylresorcin, 4-Chlorresorcin, 1,2,4-Trihydroxybenzol, 2-Amino-3-hydroxypyridin, 3-Amino-2-methylamino-6-methoxypyridin, 2,6-Dihydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxyindolin, 7-Hydroxyindolin oder Gemischen dieser Verbindungen oder deren physiologisch verträglichen Salzen.

In einer bevorzugten Ausführungsform sind die erfindungsgemäßen Mittel jedoch frei von Oxidationsfarbstoffvorprodukten.

Die optional enthaltenen zusätzlichen nichtionischen und/oder anionischen direktziehenden Farbstoffe, Entwicklerkomponenten und Kupplerkomponenten werden bevorzugt jeweils in einer Menge von 0,0001 bis 5,0 Gew.-%, vorzugsweise 0,001 bis 2,5 Gew.-%, jeweils bezogen auf das anwendungsbereite Mittel, verwendet. Dabei werden Entwicklerkomponenten und Kupplerkomponenten im Allgemeinen in etwa molaren Mengen zueinander eingesetzt. Wenn sich auch der molare Einsatz als zweckmäßig erwiesen hat, so ist ein gewisser Überschuss einzelner Oxidationsfarbstoffvorprodukte nicht nachteilig, so dass Entwicklerkomponenten und Kupplerkomponenten in einem Mol-Verhältnis von 1 zu 0,5 bis 1 zu 3, insbesondere 1 zu 1 bis 1 zu 2, stehen können.

Im Falle von Oxidationsfärbemitteln enthalten die Mittel zusätzlich ein Oxidationsmittel, bevorzugt Wasserstoffperoxid. Die Mengen an Wasserstoffperoxid entsprechen den Mengen in den erfindungsgemäßen Aufhellmitteln.

Die Mittel können weiterhin als aufhellende Färbemittel eingesetzt werden. Zur Erzielung des Aufhelleffekts enthalten die Mittel dazu Wasserstoffperoxid und/oder eines seiner festen Anlagerungsprodukte an organische oder anorganische Verbindungen.

In einer weiteren Ausführungsform ist ein erfindungsgemäßes Mittel daher dadurch gekennzeichnet, dass es zusätzlich Wasserstoffperoxid und/oder eines seiner festen Anlagerungsprodukte an organische oder anorganische Verbindungen enthält.

In einer bevorzugten Ausführungsform wird Wasserstoffperoxid selbst als wässrige Lösung verwendet. Die Konzentration einer Wasserstoffperoxid-Lösung im erfindungsgemäßen Mittel wird einerseits von den gesetzlichen Vorgaben und andererseits von dem gewünschten Effekt bestimmt; vorzugsweise werden 6 bis 12 Gew.-%ige Lösungen in Wasser verwendet. Erfindungsgemäß bevorzugte anwendungsbereite Mittel des ersten ErfindungsGegenstandds sind dadurch gekennzeichnet, dass sie, bezogen auf das Gesamtgewicht des anwendungsbereiten Mittels, 0,5 bis 20 Gew.-%, vorzugsweise 1 bis 12,5 Gew.-%, besonders bevorzugt 2,5 bis 10 Gew.-% und insbesondere 3 bis 6 Gew.-% Wasserstoffperoxid, jeweils bezogen auf das Gesamtgewicht des Mittels, enthalten.

Zur Erzielung einer verstärkten Aufhell- und Bleichwirkung kann das Mittel weiterhin mindestens ein Peroxo-Salz enthalten. Geeignete Peroxo-Salze sind anorganische Peroxoverbindungen, bevorzugt ausgewählt aus der Gruppe, gebildet aus Ammoniumperoxodisulfat, Alkalimetallperoxodisulfaten, Ammoniumperoxomonosulfat, Alkalimetallperoxomonosulfaten, Alkalimetallperoxodiphosphaten und Erdalkalimetallperoxiden. Besonders bevorzuge sind Peroxodisulfate, insbesondere Ammoniumperoxodisulfat, Kaliumperoxodisulfat und Natriumperoxodisulfat.

Die Persulfate sind jeweils in einer Menge von 0,5 bis 20 Gew.-%, vorzugsweise 1 bis 12,5 Gew.-%, besonders bevorzugt 2,5 bis 10 Gew.-% und insbesondere 3 bis 6 Gew.-%, bezogen auf das Gesamtgewicht des anwendungsbereiten Mittels, in dem erfindungsgemäßen Mittel enthalten.

Eine weitere bevorzugte Ausführungsform ist ein Mittel zum Färben und gegebenenfalls Aufhellen keratinischer Fasern, welches zusätzlich Wasserstoffperoxid, eines seiner festen Anlagerungsprodukte an organische oder anorganische Verbindungen, Ammoniumperoxodisulfat, Kaliumperoxodisulfat und/oder Natriumperoxodisulfat jeweils in einer Menge von 0,5 bis 20 Gew.-%, vorzugsweise 1 bis 12,5 Gew.-%, besonders bevorzugt 2,5 bis 10 Gew.-% und insbesondere 3 bis 6 Gew.-%, bezogen auf das Gesamtgewicht des anwendungsbereiten Mittels, enthält.

Das Mittel kann zur Verstärkung der Blondierwirkung weitere Bleichkraftverstärker enthalten, wie beispielsweise Tetraacetylethylendiamin (TAED), 1,5-Diacetyl-2,4-dioxohexahydro-1,3,5-triazin (DADHT), Tetraacetylglykoluril (TAGU), N-Nonanoylsuccinimid (NOSI), n-Nonanoyl- oder Isononanoyloxybenzolsulfonat (n- bzw. i-NOBS), Phthalsäureanhydrid, Triacetin, Ethylenglykoldiacetat und 2,5-Diacetoxy-2,5-dihydrofuran sowie Carbonatsalze bzw. Hydrogencarbonatsalze, insbesondere Ammoniumhydrogencarbonat, Ammoniumcarbonat, Natriumhydrogencarbonat, Dinatriumcarbonat, Kaliumhydrogencarbonat, Dikaliumcarbonat und Calciumcarbonat, und stickstoffhaltige, heterocyclische Bleichkraftverstärker, wie 4-Acetyl-1-methylpyridinium-p-toluolsulfonat, 2-Acetyl-1-methylpyridinium-p-toluolsulfonat, sowie N-Methyl-3,4-dihydroisochinolinium-p-toluolsulfonat.

Zur weiteren Steigerung der Aufhellung kann der erfindungsgemäßen Zusammensetzung zusätzlich mindestens eine SiO₂-Verbindung, wie Kieselsäure oder Silicate, insbesondere Wassergläser, zugesetzt sein. Es kann erfindungsgemäß bevorzugt sein, die SiO₂-Verbindungen in Mengen von 0,05 Gew.-% bis 15 Gew.-%, besonders bevorzugt in Mengen von 0,15 Gew.-% bis 10 Gew.-% und ganz besonders bevorzugt in Mengen von 0,2 Gew.-% bis 5 Gew.-%, jeweils bezogen auf die erfindungsgemäße wasserfreie Zusammensetzung, einzusetzen. Die Mengenangaben geben dabei jeweils den Gehalt der SiO₂-Verbindungen (ohne deren Wasseranteil) in den Mitteln wieder.

Die anwendungsbereiten Färbemittel können weiterhin zusätzliche Wirk-, Hilfs- und Zusatzstoffe enthalten, um die Färbeleistung zu verbessern und weitere gewünschte Eigenschaften der Mittel einzustellen.

Vorzugsweise werden die anwendungsbereiten Färbemittel als flüssige Zubereitung bereitgestellt und den Mitteln daher zusätzlich eine weitere oberflächenaktive Substanz zugesetzt, wobei solche oberflächenaktive Substanzen je nach Anwendungsgebiet als Tenside oder als Emulgatoren bezeichnet werden: Sie sind bevorzugt aus anionischen, kationischen und nichtionischen Tensiden ausgewählt.

Zusätzlich zu den amphoteren und/oder zwitterionischen Tensiden (c) können die erfindungsgemäßen Mittel weiterhin zusätzlich mindestens ein anionisches Tensid enthalten. Bevorzugte anionische Tenside sind Fettsäuren, Alkylsulfate, Alkylethersulfate und Ethercarbonsäuren mit 10 bis 20 C-Atomen in der Alkylgruppe und bis zu 16 Glykolethergruppen im Molekül. Die zusätzlichen anionischen Tenside werden in Anteilen von 0,1 bis 45 Gew.%, bevorzugt 1 bis 30 Gew.% und ganz besonders bevorzugt von 1 bis 15 Gew.%, bezogen auf die Gesamtmenge des anwendungsbereiten Mittels, eingesetzt.

Auch mindestens ein nichtionisches Tensid kann zusätzlich im erfindungsgemäßen Mittel enthalten sein. Als nichtionische Tenside eignen sich insbesondere Anlagerungsprodukte von Ethylenoxid und/oder Propylenoxid an Fettalkohole, Fettsäuren, Fettsäureamide, Polyolester von Fettsäuren sowie und Polyolether von Fettalkoholen sowie Alkylpolyglucoside. Beispiele für geeignete nichtionische Tenside sind Laureth-2, Beheneth-10, Ceteareth-12, Trideceth-12, Oleth-16, Ceteareth-20, Ceteareth-30 und Ceteareth-50 sowie PPG-1 Trideceth-6, PEG-7 Oleate, PEG-90 Stearate, PEG-30 Cocoate, Polysorbate-20, Polysorbate-60, Polysorbate-65, Polysorbate-80, Polysorbate-85, Lauryl Glucoside, Decyl Glucoside und/oder Coco Glucoside.

Die nichtionischen Tenside werden in Mengen von 0,01 bis 45 Gew.-%, bevorzugt 0,1 bis 30 Gew.-% und ganz besonders bevorzugt von 1 bis 15 Gew.-%, bezogen auf die Gesamtmenge der anwendungsbereiten Mittel, eingesetzt.

Erfindungsgemäß geeignete Mittel können auch kationische Tenside vom Typ der quartären Ammoniumverbindungen, der Esterquats und der Amidoamine enthalten. Bevorzugte quaternäre Ammoniumverbindungen sind Ammoniumhalogenide sowie die unter den INCI-Bezeichnungen Quaternium-27 und Quaternium-83 bekannten Imidazolium-Verbindungen. Weitere erfindungsgemäß verwendbare kationische Tenside stellen die quaternisierten Proteinhydrolysate dar. Eine erfindungsgemäß besonders geeignete Verbindung aus der Amidoamine stellt das unter der Bezeichnung Tegoamid^{®} S 18 im Handel erhältliche Stearamidopropyl-dimethylamin dar. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Die kationischen Tenside sind in den erfindungsgemäß verwendeten Mitteln bevorzugt in Anteilen von 0,05 bis 10 Gew.-%, bezogen auf das gesamte Mittel, enthalten.

Die anwendungsbereiten Mittel können weitere Hilfs- und Zusatzstoffe enthalten. So hat es sich als vorteilhaft erwiesen, wenn die Mittel mindestens ein Verdickungsmittel enthalten. Bezüglich dieser Verdickungsmittel bestehen keine prinzipiellen Einschränkungen. Es können sowohl organische als auch rein anorganische Verdickungsmittel zum Einsatz kommen.

Geeignete Verdickungsmittel sind
- kationische, synthetische Polymere;
- anionische, synthetische Polymere, wie Polyacrylate, Acrylates Copolymer, Copolymere von Acrylsäure und Methacrylsäure
- natürlich vorkommende Verdickungsmittel, wie nichtionische Guargums, Skleroglucangums oder Gummi arabicum, Ghatti-Gummi, Karaya-Gummi, Tragant-Gummi, Carrageen-Gummi, Johannisbrotkernmehl, Pektine, Xanthane, Alginate, Stärke-Fraktionen und Derivate wie Amylose, Amylopektin und Dextrine, sowie Cellulosederivate, wie beispielsweise Carboxymethylcellulose Methylcellulose und Hydroxyalkylcellulosen;
- nichtionische, vollsynthetische Polymere, wie Polyvinylalkohol oder Polyvinylpyrrolidinon; sowie
- anorganische Verdickungsmittel, insbesondere Schichtsilikate wie beispielsweise Bentonit, besonders Smektite, wie Montmorillonit oder Hectorit.

Färbeprozesse auf Keratinfasern laufen üblicherweise im schwach sauren bis alkalischen Bereich, bevorzugt im schwach sauren bis schwach alkalischen Milieu ab. Um die Keratinfasern und auch die Haut so weit wie möglich zu schonen, ist die Einstellung eines zu hohen pH-Wertes jedoch nicht wünschenswert. Der pH-Wert der erfindungsgemäßen Mittel kann daher zwischen 3 und 11, bevorzugt zwischen 5 und 8 liegen. Bei den pH-Werten im Sinne der vorliegenden Erfindung handelt es sich um pH-Werte, die bei einer Temperatur von 22 °C gemessen wurden.

Die zur Einstellung des bevorzugten pH-Wertes erfindungsgemäß verwendbaren Alkalisierungsmittel werden bevorzugt aus Ammoniak, Alkanolaminen, basischen Aminosäuren sowie anorganischen Alkalisierungsmitteln ausgewählt. Bevorzugte anorganische Alkalisierungsmittel sind Magnesiumcarbonat, Natriumhydroxid, Kaliumhydroxid, Natriumsilicat und Natriummetasilicat. Erfindungsgemäß einsetzbare, organische Alkalisierungsmittel werden bevorzugt ausgewählt aus Monoethanolamin, 2-Amino-2-methylpropanol und Triethanolamin. Die als erfindungsgemäßes Alkalisierungsmittel einsetzbaren basischen Aminosäuren werden bevorzugt ausgewählt aus der Gruppe, die gebildet wird aus Arginin, Lysin, Ornithin und Histidin, besonders bevorzugt Arginin.. Zur Einstellung des pH-Werts verwendbare Acificierungsmittel sind organische Säuren, wie Citronensäure, Essigsäure, Ascorbinsäure, Benzoesäure, Milchsäure, Äpfelsäure und Maleinsäure, sowie mineralische Säuren, wie Salzsäure, Schwefelsäure oder Phosphorsäure.

Weiterhin hat es sich als vorteilhaft erweisen, wenn die Färbemittel, insbesondere wenn sie zusätzlich Wasserstoffperoxid enthalten, mindestens einen Stabilisator oder Komplexbildner enthalten. Besonders bevorzugte Stabilisatoren sind Phenacetin, Alkalibenzoate (Natriumbenzoat) und Salicylsäure. Weiterhin können alle Komplexbildner des Standes der Technik eingesetzt werden. Erfindungsgemäß bevorzugte Komplexbildner sind stickstoffhaltigen Polycarbonsäuren, insbesondere EDTA und EDDS, und Phosphonate, insbesondere 1-Hydroxyethan-1,1-diphosphonat (HEDP) und/oder Ethylendiamintetramethylenphosphonat (EDTMP) und/oder Diethylentriaminpentamethylenphosphonat (DTPMP) bzw. deren Natriumsalze.

Ferner können die erfindungsgemäßen Mittel weitere Wirk-, Hilfs- und Zusatzstoffe enthalten, wie beispielsweise nichtionische Polymere wie beispielsweise Vinylpyrrolidinon/Vinylacrylat-Copolymere, Polyvinylpyrrolidinon, Vinylpyrrolidinon/Vinylacetat-Copolymere, Polyethylenglykole und Polysiloxane; zusätzliche Silikone wie flüchtige oder nicht flüchtige, geradkettige, verzweigte oder cyclische, vernetzte oder nicht vernetzte Polyalkylsiloxane (wie Dimethicone oder Cyclomethicone), Polyarylsiloxane und/oder Polyalkylarylsiloxane, insbesondere Polysiloxane mit organofunktionelle Gruppen, wie substituierten oder unsubstituierten Aminen (Amodimethicone), Carboxyl-, Alkoxy- und/oder Hydroxylgruppen (Dimethiconcopolyole), lineare Polysiloxan(A)-Polyoxyalkylen(B)-Blockcopolymere, gepfropften Silikonpolymere; kationische Polymere wie quaternisierte Celluloseether, Polysiloxane mit quaternären Gruppen, zwitterionische und amphotere Polymere, wie insbesondere Polyquaternium-22 und Polyquaternium-39; Strukturanten wie Glucose, Maleinsäure und Milchsäure, haarkonditionierende Verbindungen wie Phospholipide, beispielsweise Lecitin und Kephaline; Parfümöle, Dimethylisosorbid und Cyclodextrine; faserstrukturverbessernde Wirkstoffe, insbesondere Mono-, Di- und Oligosaccharide wie beispielsweise Glucose, Galactose, Fructose, Fruchtzucker und Lactose; Farbstoffe zum Anfärben des Mittels; Antischuppenwirkstoffe wie Piroctone Olamine, Zink Omadine und Climbazol; Aminosäuren und Oligopeptide; Proteinhydrolysate auf tierischer und/oder pflanzlicher Basis, sowie in Form ihrer Fettsäure-Kondensationsprodukte oder gegebenenfalls anionisch oder kationisch modifizierten Derivate; pflanzliche Öle; Lichtschutzmittel und UV-Blocker; Wirkstoffe wie Panthenol, Pantothensäure, Pantolacton, Allantoin, Pyrrolidinoncarbonsäuren und deren Salze sowie Bisabolol; Polyphenole, insbesondere Hydroxyzimtsäuren, 6,7-Dihydroxycumarine, Hydroxybenzoesäuren, Catechine, Tannine, Leukoanthocyanidine, Anthocyanidine, Flavanone, Flavone und Flavonole; Ceramide oder Pseudoceramide; Vitamine, Provitamine und Vitaminvorstufen; Pflanzenextrakte; Fette und Wachse wie Fettalkohole, Bienenwachs, Montanwachs und Paraffine; Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate; Trübungsmittel wie Latex, Styrol/PVP- und Styrol/Acrylamid-Copolymere; Perlglanzmittel wie Ethylenglykolmono- und - distearat sowie PEG-3-distearat; Pigmente sowie Treibmittel wie Propan-Butan-Gemische, N₂O, Dimethylether, CO₂ und Luft.

Die Auswahl dieser weiteren Stoffe wird der Fachmann gemäß der gewünschten Eigenschaften der Mittel treffen. Bezüglich weiterer fakultativer Komponenten sowie der eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen. Die zusätzlichen Wirk- und Hilfsstoffe werden in den erfindungsgemäßen Mitteln bevorzugt in Mengen von jeweils 0,0001 bis 25 Gew.-%, insbesondere von 0,0005 bis 15 Gew.-%, bezogen auf das Gesamtgewicht der Anwendungsmischung, eingesetzt.

Zur Anwendung der erfindungsgemäßen Mittel eignet sich insbesondere ein Verfahren zum Färben und gegebenenfalls Aufhellen von keratinischen Fasern, insbesondere menschlichen Haaren, welches dadurch gekennzeichnet ist, dass ein Mittel des ersten Erfindungsgegenstandds auf die keratinhaltigen Fasern aufgebracht, 5 bis 60 Minuten auf der Faser belassen und anschließend mit Wasser wieder ausgespült oder mit einem Shampoo ausgewaschen wird. Bevorzugt beträgt die Einwirkzeit der anwendungsbereiten Färbemittel 5 bis 45 min, insbesondere 10 bis 40 min, besonders bevorzugt 15 bis 35 min. Während der Einwirkzeit des Mittels auf der Faser kann es vorteilhaft sein, den Aufhellvorgang durch Wärmezufuhr zu unterstützen. Die Wärmezufuhr kann durch eine externe Wärmequelle, wie z.B. warme Luft eines Warmluftgebläses, als auch, insbesondere bei einer Haaraufhellung am lebenden Probanden, durch die Körpertemperatur des Probanden erfolgen. Bei letzterer Möglichkeit wird üblicherweise die aufzuhellende Partie mit einer Haube abgedeckt. Eine Einwirkphase bei Raumtemperatur ist ebenfalls erfindungsgemäß. Insbesondere liegt die Temperatur während der Einwirkzeit zwischen 20 °C und 40 °C, insbesondere zwischen 25 °C und 38 °C. Nach Ende der Einwirkzeit wird die verbleibende Färbezubereitung mit Wasser oder einem Reinigungsmittel aus dem Haar gespült. Als Reinigungsmittel kann dabei insbesondere handelsübliches Shampoo dienen, wobei insbesondere dann auf das Reinigungsmittel verzichtet werden kann und der Ausspülvorgang mit Wasser erfolgen kann, wenn das Färbemittel einen stark tensidhaltigen Träger besitzt.

Die erfindungsgemäßen Mittel können als Einkomponentenmittel (Färbe- und Aufhellmittel) oder als Mehrkomponentenmittel wie Zweikomponenten-Mittel oder Dreikomponenten-Mittel formuliert und entsprechend angewendet werden. Eine Auftrennung in Mehrkomponentensysteme bietet sich insbesondere dort an, wo Inkompatibilitäten der Inhaltsstoffe zu erwarten oder zu befürchten sind; das einzusetzende Mittel wird bei solchen Systemen vom Verbraucher direkt vor der Anwendung durch Vermischen der Komponente hergestellt.

Enthält das erfindungsgemäße Mittel sowohl direktziehende Farbstoffe - sowie gegebenenfalls zusätzlich Oxidationsfarbstoffvorprodukte - als auch Oxidationsmittel, so werden diese, um eine vorzeitige, unerwünschte Reaktion miteinander zu verhindern, zweckmäßigerweise getrennt voneinander konfektioniert und erst unmittelbar vor der Anwendung in Kontakt gebracht.

Ein Färbeverfahren, bei dem die Färbecreme und das Oxidationsmittel zunächst getrennt vorliegen, ist daher bevorzugt. Ein weiterer Gegenstand der vorliegenden Erfindung ist daher ein Verfahren zum Färben und Aufhellen von menschlichen Haaren, bei dem eine Zusammensetzung auf wässriger Grundlage, enthaltend Wasserstoffperoxid, mit einem erfindungsgemäßen Mittel enthaltend die wesentlichen Bestandteile (a), (b), (c) und (d) zu einer homogenen Zusammensetzung vermischt, und diese auf das Haar aufgebracht wird.

Die Formulierung enthalten eine Kombination aus
(a) mindestens ein kationisches Anthrachinonderivat der Formel (I) 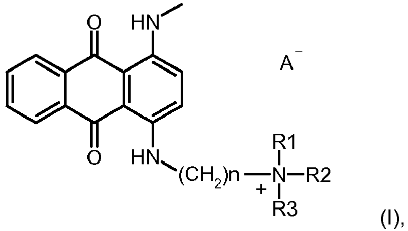 worin
   R1, R2, R3 unabhängig voneinander für eine C₁-C₆-Alkylgruppe stehen,
   n für eine ganze Zahl von 2 bis 8 steht und
   A⁻für ein physiologisch verträgliches Anion steht,
(b) mindestens einen weiteren kationischen direktziehenden Farbstoff, der von Formel (I) verschieden ist,
(c) mindestens ein amphoteres und/oder zwitterionisches Tensid und
(d) mindestens ein kationisches Polymer
eignet sich hervorragend zur Erzeugung von intensiven Färbungen mit hohem und lang anhaltendem Glanz auf keratinischen Fasern.

Ein weiterer Gegenstand der vorliegenden Erfindung ist die Verwendung eines Mittels des ersten Erfindungsgegenstands zur Erzeugung von Haarfärbungen mit erhöhtem und lang anhaltendem Glanz.

Bezüglich weiterer bevorzugter Ausführungsformen der erfindungsgemäßen Verfahren und Verwendung gilt mutatis mutandis das zu den erfindungsgemäßen Mitteln Gesagte.

### Beispiele:

Es wurden die folgenden Rezepturen hergestellt. Die Mengenangaben verstehen sich, sofern nichts anderes vermerkt ist, jeweils in Gewichtsprozent und die eingesetzte Aktivsubstanz.

| | **V1** | **V2** | **V3** | **V4** |
|---|---|---|---|---|
| Lorol C₁₂-C₁₈ techn. (C₁₂-C₁₈ Fettalkohole) | 6,0 | 6,0 | 6,0 | 6,0 |
| Lanette D (Cetearylalkohol) | 12,0 | 12,0 | 12,0 | 12,0 |
| Propylparaben | 0,19 | 0,19 | 0,19 | 0,19 |
| Methylparaben | 0,4 | 0,4 | 0,4 | 0,4 |
| Cocoamphodipropionat, Dinatriumsalz | 0,72 | 0,72 | 0,72 | 0,72 |
| Ceteareth-20 | 0,9 | 0,9 | 0,9 | 0,9 |
| Bluequat B | --- | -- | --- | --- |
| Basic Red 51 | --- | 0,2 | 0,1 | --- |
| Basic Yellow 87 | 0,2 | --- | --- | --- |
| Basic Orange 31 | 0,04 | --- | 0,2 | --- |
| Basic Violet 2 | --- | --- | --- | 0,1 |
| Polyquaternium-6 | 0,5 | 0,5 | 0,5 | 0,5 |
| Monoethanolamin | 0,02 | 0,02 | 0,02 | 0,02 |
| Hydroxyethan-1,1-diphosphonsäure | 0,012 | 0,012 | 0,012 | 0,012 |
| Wasser (dest.) | ad 100 | ad 100 | ad 100 | ad 100 |

| | **E1** | **E2** | **E3** | **E4** |
|---|---|---|---|---|
| Lorol C₁₂-C₁₈ techn. (C₁₂-C₁₈ Fettalkohole) | 6,0 | 6,0 | 6,0 | 6,0 |
| Lanette D (Cetearylalkohol) | 12,0 | 12,0 | 12,0 | 12,0 |
| Propylparaben | 0,19 | 0,19 | 0,19 | 0,19 |
| Methylparaben | 0,4 | 0,4 | 0,4 | 0,4 |
| Cocoamphodipropionat, Dinatriumsalz | 0,72 | 0,72 | 0,72 | 0,72 |
| Ceteareth-20 | 0,9 | 0,9 | 0,9 | 0,9 |
| Bluequat B | 0,4 | 0,285 | 0,15 | 0,05 |
| Basic Red 51 | 0,005 | --- | --- | --- |
| Basic Yellow 87 | --- | 0,025 | 0,15 | --- |
| Basic Orange 31 | --- | --- | --- | --- |
| Basic Violet 2 | --- | --- | --- | 0,0875 |
| Polyquaternium-6 | 0,5 | 0,5 | 0,5 | 0,5 |
| Monoethanolamin | 0,02 | 0,02 | 0,02 | 0,02 |
| Hydroxyethan-1,1-diphosphonsäure | 0,012 | 0,012 | 0,012 | 0,012 |
| Wasser (dest.) | ad 100 | ad 100 | ad 100 | ad 100 |

Der Glanz von blondierten Haarsträhnen (Fa. Kerling, 1 x blondiert mit einem Standart Blondiermittel) wurde apparativ gemessen. Dann wurden die zuvor hergestellten Färbeformulierungen auf die vermessenen Haarsträhnen (Fa. Kerling) appliziert (Flottenverhältnis 4:1, 4 g Creme pro g Haar) und für 30 Minuten einwirken gelassen. Danach wurden die Haarsträhnen ausgespült und getrocknet. Die getrockneten Haarsträhnen wurden ein zweites Mal in der Glanzapparatur vermessen.

Bei der Glanzapparatur handelt es sich um eine mit einer lichtabsorbierenden Auskleidung versehene Glanzkammer. Die zu vermessenden Haarsträhnen wurden auf einen Zylinder gespannt und in der Mitte der Glanzkammer positioniert. Oberhalb des Zylinders befindet sich als Beleuchtungsquelle eine stabförmige Gasentladungslampe, welche die auf dem Zylinder aufgespannte Haarsträhne durch eine kleine Blende beleuchtet. Mit einer Digitalkamera wurde die Glanzkurve jeder Strähne vermessen und bildanalytisch ausgewertet. Anschließend wurde der Glanzwert der Strähnen (L) nach der Formel nach Reich und Robbins (1) berechnet: ${L}_{Reich / Robbins} = \frac{S}{D * W \frac{1}{2}}$
- L:: Glanz
- S:: gerichtete Reflexion
- D:: diffuse Reflexion
- W½:: Halbwertsbreite der Glanzkurve
- (1): C. Reich and C.C. Robbins, J. Soc. Cosmet. Chem. 44, 221-234 (1993)

Die Veränderung des Glanzes wurde durch Vergleich der vor und nach der Applikation des erfindungsgemäßen Mittels gemessenen Glanzwerte bewertet. Mittels statistischer Verfahren wurde berechnet, ob die Änderung des Glanzes signifikant ist.

Bei den Messungen wurden die folgenden Glanzwert erhalten. Je höher der Glanzwert ist, desto stärker ist der Glanz

| Formulierung | Glanzwert vor der Färbung | Glanzwert nach der Färbung | Erhöhung des Glanzwertes (Quotient aus dem Glanz nach der Färbung / Glanz vor der Färbung) |
|---|---|---|---|
| V1 | 0,0017 | 0,0018 | 1,06 |
| V2 | 0,002 | 0,0075 | 3,75 |
| V3 | 0,002 | 0,0056 | 2,8 |
| V4 | 0,002 | 0,0092 | 4,6 |
| E1 | 0,002 | 0,0147 | 7,35 |
| E2 | 0,002 | 0,0143 | 7,15 |
| E3 | 0,002 | 0,0140 | 7,00 |
| E4 | 0,002 | 0,0174 | 8,7 |

## Patentansprüche

1. Mittel zum Färben von keratinischen Fasern, insbesondere menschlichen Haaren, enthaltend in einem kosmetischen Träger
(a) mindestens ein kationisches Anthrachinonderivat der Formel (I) 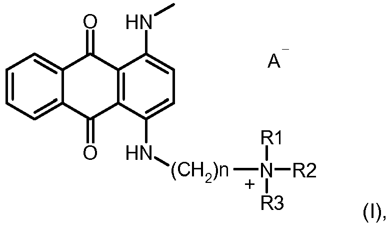 worin
R1, R2, R3 unabhängig voneinander für eine C₁-C₆-Alkylgruppe stehen,
n für eine ganze Zahl von 2 bis 8 steht und
A⁻ für ein physiologisch verträgliches Anion steht,
(b) mindestens einen weiteren kationischen direktziehenden Farbstoff, der von Formel (I) verschieden ist,
(c) mindestens ein amphoteres und/oder zwitterionisches Tensid und
(d) mindestens ein kationisches Polymer.

2. Mittel nach Anspruch 1, **dadurch gekennzeichnet**, das es mindestens ein kationisches Anthrachinonderivat (a) der Formel (I) enthält, bei dem
- R1 und R2 jeweils für eine Methylgruppe stehen,
- R3 für eine n-Propylgruppe steht und
- n für die Zahlen 2 oder 3, bevorzugt für die Zahl 3, steht.

3. Mittel nach einem der Ansprüche 1 bis 2, **dadurch gekennzeichnet, dass** es ein oder mehrere kationische Anthrachinonderivate der Formel (I) in einer Gesamtmenge von 0,0005 bis 0,5 Gew.-%, bevorzugt von 0,02 bis 0,4 Gew.-%, weiter bevorzugt von 0,05 bis 0,3 Gew.-% und insbesondere bevorzugt von 0,10 bis 0,35 Gew.-% -jeweils bezogen auf das Gesamtgewicht des Mittels - enthält.

4. Mittel nach einem der Ansprüche 1 bis 3, **dadurch gekennzeichnet, dass** es sich bei dem oder bei den kationischen direktziehenden Farbstoffen (b), die von der Formel (I) verschieden sind, um mindestens einen Farbstoff ausgewählt aus der Gruppe Basic Yellow 87, Basic Orange 31, Basic Red 51, Basic Violet 2 und Cationic Blue 347, bevorzugt aus der Gruppe Basic Yellow 87, Basic Orange 31, Basic Red 51 und Basic Violet 2, handelt.

5. Mittel nach einem der Ansprüche 1 bis 4, **dadurch gekennzeichnet, dass** es ein oder mehrere kationische direktziehende Farbstoffe (b), die von Formel (I) verschieden sind, in einer Gesamtmenge von 0,0005 bis 0,55 Gew.-%, bevorzugt von 0,005 bis 0,40 Gew.-%, weiter bevorzugt von 0,025 bis 0,30 Gew.-% und insbesondere bevorzugt von 0,05 bis 0,20 Gew.-% - jeweils bezogen auf das Gesamtgewicht des Mittels - enthält.

6. Mittel nach einem der Ansprüche 1 bis 5, **dadurch gekennzeichnet, dass** es als amphoteres und/oder zwitterionisches Tensid (c) mindestens ein Tensid aus der Gruppe der Betaine, der N-Alkyl-N,N-dimethylammonium-glycinate, der N-Acyl-aminopropyl-N,N-dimethylammoniumglycinate, der 2-Alkyl-3-carboxymethyl-3-hydroxyethyl-imidazoline, der N-Alkylglycine, der N-Alkylpropionsäuren, der N-Alkylaminobuttersäuren, der N-Alkyliminodipropionsäuren, der N-Hydroxyethyl-N-alkylamidopropylglycine, der N-Alkyltaurine, der N-Alkylsarcosine, der 2-Alkylaminopropionsäuren und der Alkylaminoessigsäuren enthält.

7. Mittel nach einem der Ansprüche 1 bis 6, **dadurch gekennzeichnet, dass** es als amphoteres und/oder zwitterionisches Tensid (c) mindestens eine Verbindung der Formel (II) und/oder der Formel (III) enthält, 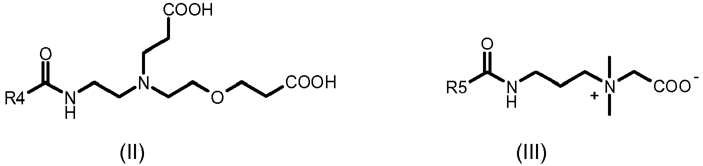 worin
R4, R5 jeweils unabhängig voneinander für eine gesättigte oder ungesättigte, verzweigte oder unverzweigte C₈-C₂₄-Alkylgruppe stehen.

8. Mittel nach einem der Ansprüche 1 bis 7, **dadurch gekennzeichnet, dass** es ein oder mehrere amphotere und/oder zwitterionische Tenside (c) in einer Gesamtmenge von 0,1 bis 4,0 Gew.-%, bevorzugt von 0,2 bis 2,5 Gew.-%, weiter bevorzugt von 0,4 bis 1,8 Gew.-% und insbesondere bevorzugt von 0,6 bis 0,9 Gew.-% -jeweils bezogen auf das Gesamtgewicht des Mittels - enthält.

9. Mittel nach einem der Ansprüche 1 bis 8, **dadurch gekennzeichnet, dass** es als kationisches Polymer (d) mindestens ein Polymer aus der Gruppe Polyquaternium-2, Polyquaternium-5, Polyquaternium-6, Polyquaternium-7, Polyquaternium-8, Polyquaternium-9, Polyquaternium-11, Polyquaternium-16, Polyquaternium-28, Polyquaternium-32, Polyquaternium-33, Polyquaternium-44, Polyquaternium-46, Polyquaternium-55 und Polyquaternium-68 enthält.

10. Mittel nach einem der Ansprüche 1 bis 9, **dadurch gekennzeichnet, dass** es ein oder mehrere kationische Polymere (d) in einer Gesamtmenge von 0,1 bis 2,0 Gew.-%, bevorzugt von 0,2 bis 1,3 Gew.-%, weiter bevorzugt von 0,3 bis 0,9 Gew.-% und insbesondere bevorzugt von 0,4 bis 0,8 Gew.-% - jeweils bezogen auf das Gesamtgewicht des Mittels - enthält.

11. Mittel nach einem der Ansprüche 1 bis 9, **dadurch gekennzeichnet, dass** es als kationisches Polymer (d) Polyquaternium-6 in einer Menge von 0,0001 bis 10 Gew.-%, bevorzugt von 0,001 bis 8 Gew.-%, weiter bevorzugt von 0,01 bis 5 Gew.-% und insbesondere bevorzugt von 0,05 bis 2,5 Gew.-% - jeweils bezogen auf das Gesamtgewicht des Mittels - enthält.

## Claims

1. An agent for dyeing keratin fibers, in particular human hair, containing, in a cosmetic carrier,
(a) at least one cationic anthraquinone derivative of formula (I) 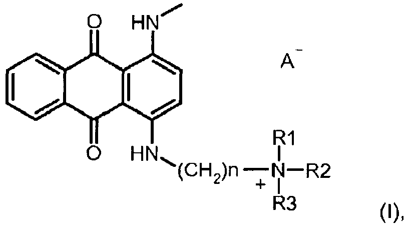 in which
R1, R2 and R3 represent, independently of one another, a C₁-C₆ alkyl group,
n represents an integer from 2 to 8, and
A⁻ represents a physiologically acceptable anion,
(b) at least one additional cationic direct dye that is different from formula (I),
(c) at least one amphoteric and/or zwitterionic surfactant and
(d) at least one cationic polymer.

2. The agent according to claim 1, **characterized in that** it contains at least one cationic anthraquinone derivative (a) of formula (I), in which
- R1 and R2 each represent a methyl group,
- R3 represents an n-propyl group and
- n represents the numbers 2 or 3, preferably the number 3.

3. The agent according to one of claims 1 to 2, **characterized in that** it contains one or more cationic anthraquinone derivatives of formula (I) in a total quantity of from 0.0005 to 0.5 wt.%, preferably from 0.02 to 0.4 wt.%, more preferably from 0.05 to 0.3 wt.%, and particularly preferably from 0.10 to 0.35 wt.%, based on the total weight of the agent in each case.

4. The agent according to one of claims 1 to 3, **characterized in that** the cationic direct dye or dyes (b) that are different from formula (I) are at least one dye selected from the group consisting of Basic Yellow 87, Basic Orange 31, Basic Red 51, Basic Violet 2 and Cationic Blue 347, preferably from the group consisting of Basic Yellow 87, Basic Orange 31, Basic Red 51 and Basic Violet 2.

5. The agent according to one of claims 1 to 4, **characterized in that** it contains one or more cationic direct dyes (b) that are different from formula (I) in a total quantity of from 0.0005 to 0.55 wt.%, preferably from 0.005 to 0.40 wt.%, more preferably from 0.025 to 0.30 wt.%, and particularly preferably from 0.05 to 0.20 wt.%, based on the total weight of the agent in each case.

6. The agent according to one of claims 1 to 5, **characterized in that** it contains, as the amphoteric and/or zwitterionic surfactant (c), at least one surfactant from the group consisting of betaines, N-alkyl-N,N-dimethylammonium glycinates, N-acyl-aminopropyl-N,N-dimethylammonium glycinates, 2-alkyl-3-carboxymethyl-3-hydroxyethyl imidazolines, N-alkyl glycines, N-alkyl propionic acids, N-alkylamino butanoic acids, N-alkylimino dipropionic acids, N-hydroxyethyl-N-alkylamido propylglycines, N-alkyl taurines, N-alkyl sarcosines, 2-alkylamino propionic acids and alkylamino acetic acids.

7. The agent according to one of claims 1 to 6, **characterized in that** it contains, as the amphoteric and/or zwitterionic surfactant (c), at least one compound of formula (II) and/or of formula (III), 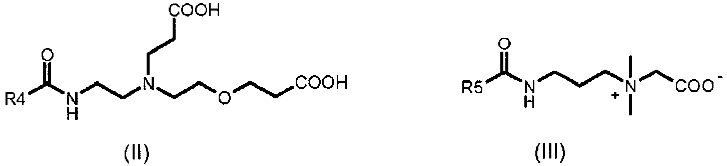 in which
R4 and R5 each represent, independently of one another, a saturated or unsaturated, branched or unbranched C₈-C₂₄ alkyl group.

8. The agent according to one of claims 1 to 7, **characterized in that** it contains one or more amphoteric and/or zwitterionic surfactants (c) in a total quantity of from 0.1 to 4.0 wt.%, preferably from 0.2 to 2.5 wt.%, more preferably from 0.4 to 1.8 wt.%, and particularly preferably from 0.6 to 0.9 wt.%, based on the total weight of the agent in each case.

9. The agent according to one of claims 1 to 8, **characterized in that** it contains, as the cationic polymer (d), at least one polymer from the group consisting of polyquaternium-2, polyquaternium-5, polyquaternium-6, polyquaternium-7, polyquaternium-8, polyquaternium-9, polyquaternium-11, polyquaternium-16, polyquaternium-28, polyquaternium-32, polyquaternium-33, polyquaternium-44, polyquaternium-46, polyquaternium-55 and polyquaternium-68.

10. The agent according to one of claims 1 to 9, **characterized in that** it contains one or more cationic polymers (d) in a total quantity of from 0.1 to 2.0 wt.%, preferably from 0.2 to 1.3 wt.%, more preferably from 0.3 to 0.9 wt.%, and particularly preferably from 0.4 to 0.8 wt.%, based on the total weight of the agent in each case.

11. The agent according to one of claims 1 to 9, **characterized in that** it contains, as the cationic polymer (d), polyquaternium-6 in a quantity of from 0.0001 to 10 wt.%, preferably from 0.001 to 8 wt.%, more preferably from 0.01 to 5 wt.%, and particularly preferably from 0.05 to 2.5 wt.%, based on the total weight of the agent in each case.

## Revendications

1. Produit de coloration de fibres de kératine, en particulier de cheveux humains, contenant dans un vecteur cosmétique :
a) au moins un dérivé cationique de l'anthraquinone de formule (I) 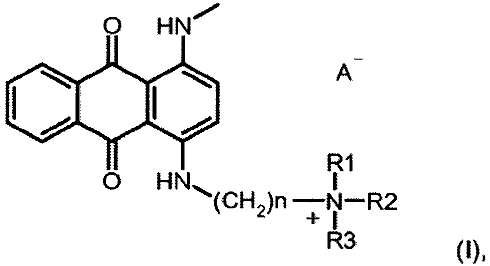 où
R1, R2, R3 représentent, indépendamment les uns des autres, un groupe C₁₋₆-alkyle,
n représente un entier entre 2 et 8, et
A⁻ représente un anion physiologiquement compatible,
b) au moins un autre colorant direct cationique, différent de la formule (I),
c) au moins un tensioactif amphotère et/ou zwitterionique, et
d) au moins un polymère cationique.

2. Produit selon la revendication 1, **caractérisé en ce qu'**il contient au moins un dérivé cationique de l'anthraquinone (a) de formule (I), pour lequel :
- R1 et R2 représentent respectivement un groupe méthyle,
- R3 représente un groupe n-propyle, et
- n = 2 ou 3, de préférence 3.

3. Produit selon une des revendications 1 ou 2, **caractérisé en ce qu'**il contient au moins un dérivé cationique de l'anthraquinone de formule (I) à hauteur de 0,0005 à 0,5 % en poids, de préférence de 0,02 à 0,4 % en poids, plus préférentiellement de 0,05 à 0,3 % en poids et en particulier de 0,10 à 0,35 % en poids, respectivement rapporté au poids total du produit.

4. Produit selon une des revendications 1 à 3, **caractérisé en ce que** la ou les colorants directs cationiques (b) différents de la formule (I) sont au moins un colorant choisis dans le groupe constitué de Basic Yellow 87, Basic Orange 31, Basic Red 51, Basic Violet 2 et Cationic Blue 347, de préférence Basic Yellow 87, Basic Orange 31, Basic Red 51 et Basic Violet 2.

5. Produit selon une des revendications 1 à 4, **caractérisé en ce qu'**il contient au moins un colorant direct cationique (b) différents de la formule (I) à hauteur de 0,0005 à 0,55 % en poids, de préférence de 0,005 à 0,40 % en poids, plus préférentiellement de 0,025 à 0,30 % en poids et en particulier de 0,05 à 0,20 % en poids, respectivement rapporté au poids total du produit.

6. Produit selon une des revendications 1 à 5, **caractérisé en ce qu'**il contient comme tensioactif amphotère et/ou zwitterionique (c) au moins un tensioactif issu du groupe constitué de la bétaïne, des N-alkyl-N,N-diméthyl glycinate ammoniums, des N-acylaminopropyl-N,N-diméthyl glycinate ammoniums, des 2-alkyl-3-carboxyméthyl-3-hydroxyéthyl-imidazolines, des N-alkylglycines, des acides N-alkylpropioniques, des acides N-alkylaminobutyriques, des acides N-alkyliminodipropioniques, des N-hydroxyéthyl-N-alkylamidopropylglycines, des N-alkyltaurines, des N-alkylsarcosines, des acides 2-alkylaminoprionioniques et des acides alkylaminoacétiques.

7. Produit selon une des revendications 1 à 6, **caractérisé en ce qu'**il contient comme tensioactif amphotère et/ou zwitterionique (c) au moins un tensioactif de formule (II) et/ou de formule (III) : 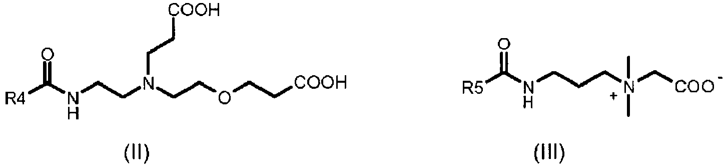 où
R4, R5 représentent respectivement, indépendamment l'un de l'autre, un groupe C₈₋₂₄-alkyle saturé ou insaturé, linéaire ou ramifié.

8. Produit selon une des revendications 1 à 7, **caractérisé en ce qu'**il contient au moins un tensioactif amphotère et/ou zwitterionique (c) à hauteur de 0,1 à 4,0 % en poids, de préférence de 0,2 à 2,5 % en poids, plus préférentiellement de 0,4 à 1,8 % en poids et en particulier de 0,6 à 0,9 % en poids, respectivement rapporté au poids total du produit.

9. Produit selon une des revendications 1 à 8, **caractérisé en ce qu'**il contient comme polymère cationique (d) au moins un polymère issu du groupe constitué de Polyquaternium 2, Polyquaternium 5, Polyquaternium 6, Polyquaternium 7, Polyquaternium 8, Polyquaternium 9, Polyquaternium 11, Polyquaternium 16, Polyquaternium 28, Polyquaternium 32, Polyquaternium 33, Polyquaternium 44, Polyquaternium 46, Polyquaternium 55 et Polyquaternium 68.

10. Produit selon une des revendications 1 à 9, **caractérisé en ce qu'**il contient au moins un polymère cationique (d) à hauteur de 0,1 à 2,0 % en poids, de préférence de 0,2 à 1,3 % en poids, plus préférentiellement de 0,3 à 0,9 % en poids et en particulier de 0,4 à 0,8 % en poids, respectivement rapporté au poids total du produit.

11. Produit selon une des revendications 1 à 9, **caractérisé en ce qu'**il contient comme polymère cationique (d) Polyquaternium 6 à hauteur de 0,0001 à 10,0 % en poids, de préférence de 0,001 à 8 % en poids, plus préférentiellement de 0,01 à 5 % en poids et en particulier de 0,05 à 2,5 % en poids, respectivement rapporté au poids total du produit.
